# EUROPÄISCHE PATENTANMELDUNG

(11) **EP 4 555 948 A1**
(43) Veröffentlichungstag der Anmeldung: **21.05.2025**
(21) Anmeldenummer: 24151780.4
(22) Anmeldetag: 15.01.2024
(51) Int. Cl.: A61B 17/29, A61B 17/22, A61M 25/00, A61M 25/09, A61B 17/221

(54) **FUNKTIONSSCHLAUCHINSTRUMENT MIT AXIALHUBBETÄTIGUNG**

(30) Priorität: 14.11.2023 DE 102023131722
(71) Anmelder: EPflex Feinwerktechnik GmbH, 72581 Dettingen/Erms (DE)
(72) Erfinder: Uihlein, Bernhard, 72581 Dettingen (DE)
(74) Vertreter: Patentanwälte Ruff, Wilhelm, Beier, Dauster & Partner mbB

(57) **Zusammenfassung**

1. Funktionsschlauchinstrument mit Axialhubbetätigung.
2.1. Die Erfindung bezieht sich auf ein Funktionsschlauchinstrument mit einem schlauchartig langgestreckten Instrumentenkörper (1), der eine biegefähige Hülle (2) und einen sich in dieser erstreckenden, biegefähigen Kern (3) aufweist und dafür eingerichtet ist, eine axiale Relativbewegung von Hülle und Kern und dadurch einen Axialhub (AH) mindestens in einem distalen Funktionsbereich (1a) zur Betätigung eines Nutzelements ermöglichen, und mit einer Bedieneinheit, mit welcher der Instrumentenkörper an einem dem distalen Funktionsbereich proximal vorgelagerten Bedienbereich (1b) zur Bewirkung des Axialhubs gekoppelt ist.
2.2. Erfindungsgemäß ist die Bedieneinheit ist dafür eingerichtet, bei Betätigung den Instrumentenkörper im Bedienbereich reversibel von einem Ausgangspositionsverlauf in einen gegenüber diesem stärker gekrümmten Krümmungsverlauf (VK) zu biegen, der eine den Axialhub bereitstellende Krümmungslängendifferenz von Hülle und Kern entlang des Krümmungsverlaufs bewirkt.
2.3. Verwendung z.B. für endoskopische Instrumente in der Medizintechnik.

## Beschreibung

Die Erfindung bezieht sich auf ein Funktionsschlauchinstrument mit einem schlauchartig langgestreckten Instrumentenkörper, der eine biegefähige Hülle und einen sich in dieser erstreckenden, biegefähigen Kern aufweist und dafür eingerichtet ist, eine axiale Relativbewegung von Hülle und Kern und dadurch einen Axialhub mindestens in einem distalen Funktionsbereich zur Betätigung eines Nutzelements ermöglichen, und mit einer Bedieneinheit, mit welcher der Instrumentenkörper an einem dem distalen Funktionsbereich proximal vorgelagerten Bedienbereich zur Bewirkung des Axialhubs gekoppelt ist.

Unter dem Begriff Hülle ist vorliegend allgemein ein beliebiges langgestrecktes Bauteil bzw. schlauchförmiges Funktionsteil zu verstehen, das einen Hohlkanal zur Durchführung des Kerns aufweist, wobei es sich je nach Anwendungsfall um ein gegenüber dem Kern biegeweicheres oder biegesteiferes Schlauch- bzw. Rohrteil oder ähnliche Umhüllung aus einem Metall- oder Kunststoffmaterial handeln kann.

Unter dem Begriff Kern ist vorliegend ein beliebiges langgestrecktes, im Hohlkanal der Hülle aufgenommenes und zumindest im distalen Funktionsbereich axial relativ zur Hülle bewegliches, drahtförmiges Bauteil bzw. Funktionsbauteil aus einem hohlen oder massiven Metall- oder Kunststoffmaterial zu verstehen.

Unter den Begriffen distaler Funktionsbereich und diesem proximal vorgelagerter Bedienbereich sind vorliegend allgemein zwei axial beabstandete oder aufeinanderfolgende Abschnitte des schlauchartig langgestreckten Instrumentenkörpers zu verstehen, von denen in einer Vorwärtsrichtung, d.h. distalen Richtung, der Bedienbereich hinter dem Funktionsbereich liegt. In typischen Fällen, jedoch nicht zwingend, befinden sich der Bedienbereich in einem hinteren Endbereich des Instrumentenkörpers und der Funktionsbereich in einem vorderen Endbereich des Instrumentenkörpers.

Funktionsschlauchinstrumente dieser Art sind beispielsweise in der endoskopischen Medizintechnik gebräuchlich, speziell in Form von Steinfangkorbinstrumenten mit entfaltbarem Drahtkorb zum Einfangen und/oder Entfernen von Steinen, thrombotischen Blutgerinnseln und dergleichen in Gewebehohlräumen sowie in Form von ähnlichen Instrumenten, wie Drahtfilterinstrumenten, Drahtschlingeninstrumenten und Fangnetzinstrumenten. Bei diesen genannten Anwendungen befindet sich im distalen Funktionsbereich, oftmals speziell am distalen Ende des Instrumentenkörpers, ein typischerweise entfaltbares Nutzelement, wie ein Drahtkorb, ein Drahtfilter, eine Drahtschlinge oder ein Fangnetz, und durch axiales Zurück- und Vorbewegen des Kerns relativ zur den Kern umgebenden Hülle wird dieses Element in das distale Ende der Hülle unter Zusammenfalten eingezogen bzw. unter Entfalten herausbewegt.

Eine weitere Anwendung sind Führungsdrahteinheiten für Katheterinstrumente, wobei es sich in diesem Fall beim Kern oftmals um einen sogenannten Zugdraht, in anderen Fällen auch um einen Schubdraht, der Schubkräfte übertragen kann, und bei der Hülle um einen diesen umgebenden Schlauch handelt, der im distalen Bereich mit dem Zugdraht verbunden ist. Durch axiale Relativbewegung von Zugdraht und Schlauch kann ein in diesem Fall als Nutzelement fungierender distaler Abschnitt der Führungsdrahteinheit bzw. des schlauch-/rohrförmigen Katheters in einer gewünschten Weise verformt, z.B. gebogen werden. Dabei wird der deshalb so bezeichnete Zugdraht typischerweise auf Zug belastet, d.h. er überträgt eine Zugkraft von der Bedieneinheit zum distalen Nutzelement. Noch eine weitere endoskopische Anwendung sind Instrumente, die im distalen Funktionsbereich ein Zangenelement oder ein Scherenelement als Nutzelement aufweisen und z.B. für Biopsieanwendungen benutzt werden. Auch in diesem Fall kann der Kern beispielsweise durch einen Zugdraht gebildet sein.

Üblicherweise umfasst die Bedieneinheit bei diesen Instrumenten in herkömmlichen Ausführungen zwei axial relativbeweglich miteinander gekoppelte Bedienteile, wobei das eine Bedienteil eine Fixierung für die Hülle aufweist und das andere Bedienteil eine Fixierung für den Kern aufweist. Die Fixierung kann z.B. durch eine übliche Formschluss- und/oder Kraftschlussverbindung erfolgen. Die Axialhubbewegung für das distale Nutzelement wird vom Benutzer durch Betätigen der Bedieneinheit bewirkt, indem er die beiden Betätigungsteile axial relativ zueinander bewegt bzw. verschiebt. Derartige Instrumente sind z.B. in den Offenlegungsschriften US 2005/0113862 A1, DE 10 2010 010 798 A1, WO 2010/133245 A1, WO 2011 /095233 A1 und DE 10 2017 210 534 A1.

Bei dieser herkömmlichen Auslegung wird die axiale Relativbewegung der beiden Bedienteile durch den Benutzer über den Instrumentenkörper als entsprechender Axialhub für die Betätigung des Nutzelements zum distalen Funktionsbereich übertragen. Für Anwendungen, bei denen nur ein relativ geringer Axialhub benötigt wird bzw. zulässig ist, beispielsweise mit einer Hublänge in der Größenordnung von nur einem oder einigen wenigen Millimetern oder weniger als 1 mm, bedingt dies eine entsprechend feinfühlige Handhabung der Bedieneinheit durch den Benutzer. Insbesondere auch in solchen Fällen kann eine exakte Einstellung einer gewünschten Axialhublänge über die Bedienheit für den Benutzer schwierig sein.

Der Erfindung liegt als technisches Problem die Bereitstellung eines Funktionsschlauchinstruments der eingangs genannten Art zugrunde, das gegenüber dem oben erwähnten Stand der Technik fertigungstechnische und/oder bedientechnische und/oder funktionelle Vorteile insbesondere im Hinblick auf die Bereitstellung des zur Betätigung des Nutzelements erforderlichen Axialhubs bietet, vor allem auch in Anwendungen mit vergleichsweise geringer benötigter Hublänge.

Die Erfindung löst dieses Problem durch die Bereitstellung eines Funktionsschlauchinstruments mit den Merkmalen des Anspruchs 1. Vorteilhafte Weiterbildungen der Erfindung sind Gegenstand der Unteransprüche, deren Wortlaut hiermit durch Verweis zum Bestandteil der Beschreibung gemacht wird. Dies schließt insbesondere auch alle Ausführungsformen der Erfindung ein, die sich aus den Merkmalskombinationen ergeben, die durch die Rückbezüge in den Unteransprüchen definiert sind.

Beim erfindungsgemäßen Funktionsschlauchinstrument ist die Bedieneinheit dafür eingerichtet, bei Betätigung den Instrumentenkörper im Bedienbereich reversibel von einem Ausgangspositionsverlauf in einen gegenüber diesem stärker gekrümmten Krümmungsverlauf zu biegen, der eine den Axialhub bereitstellende Krümmungslängendifferenz von Hülle und Kern entlang des Krümmungsverlaufs bewirkt.

Folglich sorgt die Bedieneinheit bei Betätigung durch den Benutzer dafür, dass sich der Verlauf des Instrumentenkörpers im Bedienbereich zwischen dem Ausgangspositionsverlauf und dem stärker gekrümmten Krümmungsverlauf ändert, d.h. der Instrumentenkörper kann wiederholt in seinem Verlauf von dem Ausgangspositionsverlauf in Richtung des stärker gekrümmten Krümmungsverlauf und umgekehrt von diesem Krümmungsverlauf in den schwächer gekrümmten Ausgangspositionsverlauf verändert werden, vorzugsweise stufenlos. Die Begriffe Ausgangspositionsverlauf und stärker gekrümmter Krümmungsverlauf sind demgemäß ohne Beschränkung der Allgemeinheit als die beiden gegensätzlichen Endlagen des vom Benutzer über die Bedieneinheit veränderbaren Verlaufs des Instrumentenkörpers im Bedienbereich zu verstehen. Der Ausgangspositionsverlauf des Instrumentenkörpers kann je nach Bedarf und Anwendungsfall geradlinig sein oder bereits einen gekrümmten Verlauf besitzen, der insgesamt schwächer gekrümmt ist als der für die Bereitstellung des gewünschten Axialhubs benötigte Krümmungsverlauf.

Die sich verändernde Krümmung des Instrumentenkörpers im Bedienbereich hat eine Krümmungslängendifferenz von Hülle und Kern im Bedienbereich zur Folge, die wiederum zu einer relativen axialen Verschiebung von Hülle und Kern im distalen Funktionsbereich und damit dort zur Bereitstellung des gewünschten Axialhubs führt. Grund für die Krümmungslängendifferenz ist die geometrische Eigenschaft, dass die Länge einer gekrümmten Bahn mit größer werdendem radialem Abstand vom Krümmungsmittelpunkt zunimmt.

Der Benutzer bewirkt folglich beim erfindungsgemäßen Funktionsschlauchinstrument den Axialhub zur Betätigung des Nutzelements im distalen Funktionsbereich mittels Änderung des Krümmungsverlaufs des Instrumentenkörpers im Bedienbereich durch entsprechende Betätigung der Bedieneinheit. Durch entsprechende Dimensionierung des Instrumentenkörpers und insbesondere seiner Hülle und seines Kerns lässt sich mit diesem Betätigungsmechanismus eine vergleichsweise feinfühlige Einstellung des Axialhubs für die distale Nutzelementbetätigung erzielen, d.h. es lässt sich ein relativ großer Bedienweg für den Benutzer an der Bedieneinheit realisieren, der im Sinn einer Untersetzung zu einem Axialhub mit gegenüber diesem Bedienweg deutlich geringerer Hublänge führen kann. Zudem lässt sich das dergestalt ausgeführte Instrument mit vergleichsweise geringem Aufwand fertigen, und die Bedieneinheit, die diesen Bedienmechanismus bereitstellt, lässt sich vom Benutzer sehr komfortabel und funktionssicher bedienen.

Vorzugsweise ist der Kern in einem dem Bedienbereich proximal vorgelagerten oder im Bedienbereich selbst liegenden Fixierbereich gegenüber der Hülle axial bewegungsgesichert, d.h. er ist dort an der Hülle, z.B. durch Reibschluss, oder zusammen mit dieser an einem dritten Bauteil dergestalt fixiert, dass er sich nicht oder jedenfalls nicht in dem Maß axial relativ zur Hülle bewegen kann, dass damit die Krümmungslängendifferenz ausgeglichen werden könnte, weshalb sich die Krümmungslängendifferenz zwischen Kern und Hülle ungemindert oder zumindest teilweise als Axialhub auf den distalen Funktionsbereich überträgt.

In der Regel setzt die Bedieneinheit, wenn sie vom Benutzer zur Bereitstellung des Axialhubs betätigt wird, den Instrumentenkörper und speziell dessen Hülle und Kern durch die stärkere Krümmung unter Zugspannung, da sich durch die zunehmende Krümmungslänge die Länge des Instrumentenkörpers im Bedienbereich erhöht. Mit anderen Worten fungiert die Bedieneinheit dann als eine Art Spanneinheit für den Instrumentenkörper. Dies ermöglicht es in entsprechenden Fällen, dass der Instrumentenkörper von selbst wieder vom stärker gekrümmten Verlauf in seinen Ausgangspositionsverlauf zurückkehrt, wenn der Benutzer die Bedieneinheit loslässt.

Im Fall von endoskopischen Funktionsschlauchinstrumenten kann das Nutzelement insbesondere ein biegefähiger distaler Endbereich einer Führungsdrahteinheit sein, dessen Bewegung zwischen zwei Endlagen unterschiedlicher Krümmung eine zugehörige Nutzfunktion erfüllt, oder ein z.B. als Fangkorb fungierender, auffaltbarer Drahtkorb oder ein auffaltbares Drahtfilterelement oder ein Zangen- oder Scherenelement oder eine optische Linse oder Kamera oder ein anderes in der Endoskopietechnik üblicherweise verwendetes Nutzelement.

Als weiterer Vorteil ist anzumerken, dass sich das erfindungsgemäße Funktionsschlauchinstrument aufgrund dieses Aufbaus und dieser Art der Bereitstellung des Axialhubs insbesondere derart realisieren lässt, dass der Benutzer vor betriebsgemäßem Gebrauch des Instruments den Instrumentenkörper an der Bedieneinheit nicht gegen Axialbewegung gesichert fixieren muss, sondern es genügt, dass er den Instrumentenkörper lose mit der Bedieneinheit koppelt, z.B. mittels einfachem Hindurchführen durch ein Gehäuse der Bedieneinheit. Das kann die Handhabung vereinfachen und den Nutzerkomfort erhöhen.

In einer Weiterbildung der Erfindung beinhaltet die Hülle einen Schraubenfederkörper aus einem Runddrahtmaterial oder einem Flachdrahtmaterial. Dies stellt eine vorteilhafte Realisierungsmöglichkeit für die Hülle dar. Als Runddrahtmaterial bzw. Flachdrahtmaterial kann je nach Bedarf und Anwendungsfall ein Metallmaterial oder ein Kunststoffmaterial verwendet werden. Der Schraubenfederkörper stellt bei Biegung an der radial inneren Seite eine gewünschte Längenstabilität bereit, während er sich an seiner radialen Außenseite problemlos dehnen kann. In alternativen Ausführungen kann die Hülle auch aus einem dehnfähigen, elastischen, massiven Hüllwandmaterial, i.e. Schlauchmaterial, aus Metall oder Kunststoff bestehen.

In einer Weiterbildung der Erfindung beinhaltet der Kern einen Massivdrahtkörper oder einen Hohldrahtkörper. Unter dem Begriff Massivdrahtkörper ist dabei vorliegend allgemein ein drahtförmiges Element aus Vollmaterial zu verstehen, je nach Bedarf und Anwendungsfall ein entsprechendes Metallmaterial oder Kunststoffmaterial. In alternativen Ausführungen kann der Kern einen Hohldrahtkörper beinhalten, d.h. ein drahtförmiges Element aus einem Hohlmaterial, z.B. einem Schraubenfedermaterial oder einem massiven Schlauchwandmaterial, wiederum je nach Bedarf und Anwendungsfall aus Metall oder Kunststoff. Dies eignet sich beispielsweise für Katheterinstrumente. Über den Hohlkanal des Kerns kann z.B. ein Medikament oder Kontrastmittel oder eine Nadel, ein Mandrell oder ähnliches Funktionselement eingeführt werden.

In einer Weiterbildung der Erfindung umfasst der Krümmungsverlauf einen oder mehrere bogenförmige Abschnitte. Bogenförmige Abschnitte zur Erhöhung der Krümmungslänge sind konstruktiv und funktionell vorteilhaft. Je nach geforderter Hublänge des Axialhubs können der oder die bogenförmigen Abschnitte mit entsprechender Bogenlänge gebildet werden. Die Bogenform kann geeignet gewählt sein. Von Vorteil ist im Allgemeinen eine Kreisbogenform, alternativ können der oder die Abschnitte jedoch z.B. auch ellipsenförmig bzw. ovalförmig sein oder eine unregelmäßig gebogene Form besitzen.

In einer Weiterbildung der Erfindung umfasst der Krümmungsverlauf einen oder mehrere Vollwicklungen. Diese Maßnahme eignet sich für Anwendungen, die eine entsprechend etwas größere Axialhublänge erfordern. Jede Vollwicklung, d.h. jeder vollständige Umlauf des Krümmungsverlaufs um 360°, stellt einen zugehörigen Teil der benötigten Axialhublänge bereit. Anders gesagt, durch entsprechend viele Vollwicklungen des Krümmungsverlaufs lässt sich jede gewünschte Axialhublänge innerhalb eines in der Praxis für Funktionsschlauchinstrumente benötigten Bereichs realisieren.

In einer Weiterbildung der Erfindung weist die Bedieneinheit in einem distalen Endbereich eine die Hülle gegen Axialbewegung sichernde Hüllenfixierung auf. Dies vermeidet, dass das Krümmen des Instrumentenkörpers im Bedienbereich eine entsprechende Veränderung der axialen Position der Hülle im distalen Funktionsbereich verursacht, und erleichtert folglich die Positionierung des Nutzelementes am gedachten Einsatzort. Die für das Krümmen benötigte Zusatzlänge des Instrumentenkörpers im Bedienbereich wird in diesem Fall vom proximal vorgelagerten Teil des Instrumentenkörpers nachgeliefert. Dies gilt sowohl für die Hülle als auch für den Kern, wenn dieser, wie oben erwähnt, im Fixierbereich gegenüber der Hülle axial bewegungsgesichert fixiert ist. Wie gewünscht, überträgt sich dann nur die durch das Krümmen zusätzlich bewirkte Krümmungslängendifferenz zwischen Kern und Hülle als Axialhub auf den distalen Funktionsbereich des Instrumentenkörpers.

In einer Weiterbildung der Erfindung weist die Bedieneinheit mindestens einen bewegten Biegekörper auf, gegen dessen Umfang sich der Instrumentenkörper in seinem Krümmungsverlauf anlegt. Dies stellt eine fertigungstechnisch und funktionell sehr vorteilhafte Realisierung für die Bedieneinheit dar. Bei Betätigung der Bedieneinheit bewegt der Benutzer in diesem Fall den bewegten Biegekörper, wodurch der Biegekörper den Instrumentenkörper von seinem Ausgangspositionsverlauf in den gewünschten Krümmungsverlauf biegt bzw. umgekehrt zulässt, dass dieser wieder in seinen Ausgangspositionsverlauf zurückkehrt. Die Form des Umfangs des Biegekörpers kann abhängig vom gewünschten Krümmungsverlauf des sich dagegen anlegenden Bereichs des Instrumentenkörpers geeignet gewählt werden, z.B. als im Querschnitt kreisrunde oder elliptische oder ovale Umfangsfläche, wobei der Biegekörper insgesamt z.B. als zylinderförmiger Körper mit der entsprechenden Querschnittsform ausgebildet sein kann.

Es sei an dieser Stelle angemerkt, dass der Begriff bewegt vorliegend allgemein in einem relativen Sinn zu verstehen ist, d.h. als relative Bewegung, hier des bewegten Biegekörpers gegenüber einem anderen Teil der Bedieneinheit und insbesondere auch gegenüber dem Instrumentenkörper. Dazu braucht nicht zwingend nur der bewegte Biegekörper selbst vom Benutzer aktiv bewegt werden, es kann alternativ auch vorgesehen sein, dass der Benutzer anstelle oder zusätzlich zu dem bewegten Biegekörper den anderen Teil der Bedieneinheit und mit diesem auch den Instrumentenkörper im Bedienbereich relativ zum bewegten Biegekörper aktiv bewegt und dadurch für die Relativbewegung des bewegten Biegekörper gegenüber dem Instrumentenkörper sorgt.

In einer Ausgestaltung der Erfindung ist der bewegte Biegekörper um seine Längsachse drehbeweglich und wickelt den Instrumentenkörper zum Verbringen in seinen Krümmungsverlauf auf. In dieser Realisierung fungiert der Biegekörper folglich als eine Art Wickelkern zum Auf- und Abwickeln des Instrumentenkörpers, wodurch der Instrumentenkörper den stärkeren Krümmungsverlauf einnimmt bzw. wieder in seinen Ausgangspositionsverlauf zurückkehrt.

In einer anderweitigen Ausgestaltung der Erfindung läuft der bewegte Biegekörper auf einer Umlaufbahn um und nimmt den Instrumentenkörper zum Verbringen in seinen Krümmungsverlauf mit. In dieser Ausführung fungiert der Biegekörper als eine Art Mitnahmekörper, der bei seinem Umlauf auf der Umlaufbahn den Instrumentenkörper mitnimmt und dadurch in seinen gewünschten Krümmungsverlauf biegt. Dies stellt eine funktionell und konstruktiv vorteilhafte Maßnahme dar. Die Umlaufbahn kann z.B. eine Kreisbahn, eine elliptische oder ovale Bahn etc. sein.

In weiterer Ausgestaltung der Erfindung erstreckt sich die Umlaufbahn um einen zentrischen Biegekörper herum, gegen dessen Umfang sich der Instrumentenkörper in seinem Krümmungsverlauf anlegt. Vorteilhaft trägt bei dieser Realisierung zusätzlich der zentrische Biegekörper dazu bei, den Instrumentenkörper in seinen gewünschten Krümmungsverlauf zu biegen. Dies ist für entsprechende Anwendungsfälle funktionstechnisch und konstruktiv vorteilhaft.

In anderweitiger Ausgestaltung der Erfindung weist die Bedieneinheit einen ersten und einen zweiten bewegten Biegekörper auf, gegen deren Umfang sich der Instrumentenkörper in seinem Krümmungsverlauf anlegt und die mit umlaufseitigem Abstand, d.h. mit einem Umlaufwinkelabstand, auf der Umlaufbahn umlaufen und den Instrumentenkörper zum Verbringen in seinen Krümmungsverlauf mitnehmen. Damit lässt sich für entsprechende Anwendungen bei gleichem Umlaufwinkel der Biegekörper eine größere Hublänge des Axialhubs für das distale Nutzelement bereitstellen als in Ausführungen mit nur einem umlaufenden Biegekörper.

In alternativen anderweitigen Ausgestaltungen der Erfindung weist die Bedieneinheit mindestens einen ersten und einen zweiten bewegten Biegekörper auf, die translationsbeweglich mit unterschiedlichem Abstand quer zur Verschiebungsrichtung oder schwenkbeweglich mit radialem Abstand zur Schwenkachse angeordnet sind.

In weiterer Ausgestaltung der Erfindung liegen sich der erste und der zweite Biegekörper diametral auf der Umlaufbahn gegenüber und führen zwischen sich den Instrumentenkörper. Diese Ausführung ermöglicht vorteilhaft eine vergleichsweise kompakte Bauform für die Bedieneinheit bei gegebener, geforderter Axialhublänge.

In alternativer weiterer Ausgestaltung der Erfindung sind der erste und der zweite Biegekörper um einen Umlaufbahnwinkel kleiner als 180°, insbesondere zwischen 80° und 100°, voneinander beabstandet und laufen um den zentrischen Biegekörper um. Diese Ausführung ermöglicht die Bereitstellung etwas größerer Axialhublängen bei relativ gering bleibendem Umlaufwinkel der auf der Umlaufbahn bewegten Biegekörper.

In einer anderweitigen Ausgestaltung der Erfindung ist der bewegte Biegekörper um eine Schwenkachse verschwenkbar ist und nimmt den Instrumentenkörper zum Verbringen in seinen Krümmungsverlauf mit. Die Bedieneinheit kann in diesem Fall z.B. zwei relativ zueinander schwenkbewegliche Scherenteile beinhalten, wobei der bewegte Biegekörper auf einem der beiden Scherenteile angeordnet ist.

In noch einer anderweitigen Ausgestaltung der Erfindung ist der bewegte Biegekörper längs einer Verschiebungsrichtung translationsbeweglich und nimmt den Instrumentenkörper zum Verbringen in seinen Krümmungsverlauf mit. Dazu kann die Bedieneinheit z.B. zwei relativ zueinander translationsbewegliche Bedienteile oder speziell Gehäuseteile umfassen, wobei der bewegte Biegekörper an einem der beiden Gehäuseteile angeordnet ist.

In weiterer Ausgestaltung der Erfindung weist die Bedieneinheit zwei gegeneinander verschwenkbare oder translatorisch bewegbare Bedienteile auf, an denen jeweils ein oder mehrere Biegekörper angeordnet sind, wobei zwischen zwei Biegekörpern des einen Bedienteils ein Zwischenraum zum Eingreifen eines Biegekörpers des anderen Bedienteils belassen ist. Auf diese Weise kann beim Betätigen der Bedieneinheit ein am einen Bedienteil angeordneter Biegekörper in den Zwischenraum zwischen zwei benachbarten Biegekörpern am anderen Bedienteil bewegt werden und dabei den Instrumentenkörper von seinem z.B. geradlinigen Ausgangspositionsverlauf in seinen stärker gekrümmten Krümmungsverlauf krümmen bzw. biegen. Dazu können auch die Biegekörper am anderen Bedienteil beitragen. Die so realisierte Bedieneinheit lässt sich relativ kompakt bauen und bequem handhaben.

In einer weiteren Ausgestaltung der Erfindung weist die Bedieneinheit ein Bediengehäuse mit einem Gehäuseinnenraum auf, das den mindestens einen bewegten Biegekörper und den Bedienbereich des Instrumentenkörpers aufnimmt, wobei das Bediengehäuse eine Eintrittsöffnung in den Gehäuseinnenraum und eine Austrittsöffnung aus dem Gehäuseinnenraum für den Instrumentenkörper aufweist. Dies stellt eine fertigungstechnisch, funktionell und hinsichtlich Bedienkomfort sehr vorteilhafte Realisierung der Bedieneinheit dar. Im Bediengehäuse befinden sich der oder die Biegekörper, die den Instrumentenkörper in seinem Bedienbereich in den gewünschten Krümmungsverlauf biegen, sowie ggf. die Umlaufbahn für den oder die Biegekörper. Der Instrumentenkörper wird dazu mit seinem Bedienbereich im Bediengehäuse angeordnet, wobei er über die Eintrittsöffnung in das Bediengehäuse eintritt und dieses über die Austrittsöffnung wieder verlässt.

In weiterer Ausgestaltung der Erfindung umfasst das Bediengehäuse zwei relativ zueinander drehbewegliche oder schwenkbewegliche oder translationsbewegliche Gehäuseteile, wobei die Eintrittsöffnung und die Austrittsöffnung am einen Gehäuseteil und der mindestens eine bewegte Biegekörper am anderen Gehäuseteil angeordnet sind. Diese Ausführung der Bedieneinheit ist mit relativ geringem Aufwand realisierbar und ermöglicht komfortabel und funktionssicher den gewünschten Bedienmechanismus, d.h. die Krümmungsänderung des Instrumentenkörpers im Bedienbereich von seinem Ausgangspositionsverlauf in seinen stärker gekrümmten Krümmungsverlauf.

Vorteilhafte Ausführungsformen der Erfindung sind in den Zeichnungen dargestellt. Diese und weitere Ausführungsformen der Erfindung werden nachfolgend näher erläutert. Hierbei zeigen:
- Fig. 1: eine Längsschnittansicht eines gekrümmten Bereichs eines Instrumentenkörpers eines Funktionsschlauchinstruments mit einem Schraubenfederkörper aus Runddrahtmaterial als Hülle,
- Fig. 2: die Ansicht von Fig. 1 für eine Variante mit einem Schraubenfederkörper aus Flachdrahtmaterial als Hülle,
- Fig. 3: die Ansicht von Fig. 1 für eine Variante mit einem Kern kleineren Durchmessers,
- Fig. 4: die Ansicht von Fig. 2 für eine Variante mit einem Kern kleineren Durchmessers,
- Fig. 5A: die Ansicht von Fig. 3 für eine Variante mit einer Hülle aus massivem Schlauchwandmaterial,
- Fig. 5B: die Ansicht von Fig. 5A für eine Variante mit einem Kern aus Hohlmaterial,
- Fig. 5C: die Ansicht von Fig. 4 für eine Variante mit Abstandshülse,
- Fig. 6: eine Längsschnittansicht des Instrumentenkörpers von Fig. 1 in einem Bereich mit Kernverjüngung,
- Fig. 7: die Ansicht von Fig. 6 für eine Variante mit mehrteiliger Hülle aus Flach- und Runddrahtmaterial,
- Fig. 8: eine Längsschnittansicht eines Instrumentenkörpers entsprechend Fig. 1 von einem proximalen Ende bis zu einem distalen Funktionsbereich in einem Ausgangspositionsverlauf,
- Fig. 9: die Ansicht von Fig. 8 mit dem Instrumentenkörper in einem gekrümmten Verlauf in einem Bedienbereich,
- Fig. 10: eine Längsschnittansicht eines Instrumentenkörpers entsprechend Fig. 1 von einem proximalen Ende bis zu einem distalen Funktionsbereich mit dem Instrumentenkörper in einem Krümmungsverlauf mit mindestens einer Vollwicklung in einem Bedienbereich,
- Fig. 11: eine gegenüber Fig. 10 um 90° versetzte Längsschnittansicht mit dem Instrumentenkörper in einem Krümmungsverlauf mit vier Vollwicklungen im Bedienbereich,
- Fig. 12: die Ansicht von Fig. 10 für eine Variante mit einer Drahtkorbstruktur als axialhubbetätigtes Nutzelement in einem distalen Endbereich,
- Fig. 13: eine Längsschnittansicht eines Instrumentenkörpers eines Funktionsschlauchinstruments mit einer Drahtkorbstruktur als axialhubbetätigtes Nutzelement in einem distalen Funktionsbereich mit axialem Abstand zu einem distalen Endbereich,
- Fig. 14: eine Längsschnittansicht einer Bedieneinheit mit einem zentrischen Biegekörper und einem um diesen auf einer auf ca. 270° begrenzten Umlaufbahn umlaufend bewegten Biegekörper,
- Fig. 15: eine Querschnittansicht der Bedieneinheit von Fig. 14,
- Fig. 16: die Ansicht von Fig. 15 mit in der Bedieneinheit aufgenommenem Bedienbereich eines Instrumentenkörpers in Ausgangsposition, zum leichteren Erkennen der hier interessierenden Merkmale ohne Schraffur der geschnittenen Bereiche,
- Fig. 17: die Ansicht von Fig. 16 in einer um 90° gedrehten Stellung des umlaufend bewegten Biegekörpers,
- Fig. 18: die Ansicht von Fig. 16 in einer um 135° gedrehten Stellung des umlaufend bewegten Biegekörpers,
- Fig. 19: die Ansicht von Fig. 16 für eine Variante der Bedieneinheit mit vollständig umlaufender Umlaufbahn,
- Fig. 20: die Ansicht von Fig. 19 in einer um 90° gedrehten Stellung des umlaufend bewegten Biegekörpers,
- Fig. 21: die Ansicht von Fig. 19 in einer um 270° gedrehten Stellung des umlaufend bewegten Biegekörpers,
- Fig. 22: die Ansicht von Fig. 19 in einer um 360° gedrehten Stellung des umlaufend bewegten Biegekörpers,
- Fig. 23: die Ansicht von Fig. 19 für eine Variante der Bedieneinheit mit zwei um 90° versetzt auf der Umlaufbahn umlaufenden Biegekörpern,
- Fig. 24: die Ansicht von Fig. 23 in einer um 90° gedrehten Stellung der umlaufend bewegten Biegekörper,
- Fig. 25: die Ansicht von Fig. 23 in einer um 270° gedrehten Stellung der umlaufend bewegten Biegekörper,
- Fig. 26: die Ansicht von Fig. 23 in einer um 360° gedrehten Stellung der umlaufend bewegten Biegekörper,
- Fig. 27: die Ansicht von Fig. 16 für eine Variante der Bedieneinheit mit einem als Wickelkörper fungierenden Biegekörper,
- Fig. 28: die Ansicht von Fig. 19 für eine Variante der Bedieneinheit mit zwei diametral gegenüberliegend auf der Umlaufbahn umlaufenden Biegekörpern ohne zentrischen Biegekörper,
- Fig. 29: die Ansicht von Fig. 28 in einer um 90° gedrehten Stellung der Biegekörper,
- Fig. 30: die Ansicht von Fig. 28 in einer um 180° gedrehten Stellung der Biegekörper,
- Fig. 31: die Ansicht von Fig. 28 in einer um 270° gedrehten Stellung der Biegekörper,
- Fig. 32: die Ansicht von Fig. 28 in einer um 360° gedrehten Stellung der Biegekörper,
- Fig. 33: die Ansicht von Fig. 28 für eine Variante mit Umdrehungszähler,
- Fig. 34: eine schematische Seitenansicht eines Teils einer Bedieneinheit mit axial versetzt und translatorisch quer bewegten Biegekörpern sowie distal endseitiger Klemmstift-Hüllenfixierung,
- Fig. 35: die Ansicht von Fig. 34 für eine Variante der Bedieneinheit mit Querversatz zweier der axial versetzten Biegekörper,
- Fig. 36: zwei Ansichten entsprechend Fig. 34 für eine Variante der Bedieneinheit mit drehfester Halterung eines distal endseitigen Biegekörpers als Hüllenfixierung und drehbeweglicher Lagerung der übrigen Biegekörper in einer Ausgangsposition bzw. einer Betätigungsposition,
- Fig. 37: eine Längsschnittansicht einer Bedieneinheit mit axial versetzt und translatorisch quer bewegten Biegekörpern sowie distal endseitiger Klemmstift-Hüllenfixierung in einer Ausgangsposition,
- Fig. 38: die Ansicht von Fig. 37 mit der Bedieneinheit in einer Betätigungsposition,
- Fig. 39: eine Schnittansicht längs einer Linie L39-L39 von Fig. 37,
- Fig. 40: die Schnittansicht von Fig. 39 mit der Bedieneinheit in der Betätigungsposition,
- Fig. 41: eine schematische Seitenansicht entsprechend Fig. 34 für eine Variante der Bedieneinheit mit radial versetzt und schwenkbeweglich angeordneten Biegekörpern in einer Ausgangsposition und
- Fig. 42: die Ansicht von Fig. 41 mit der Bedieneinheit in einer Betätigungsposition.

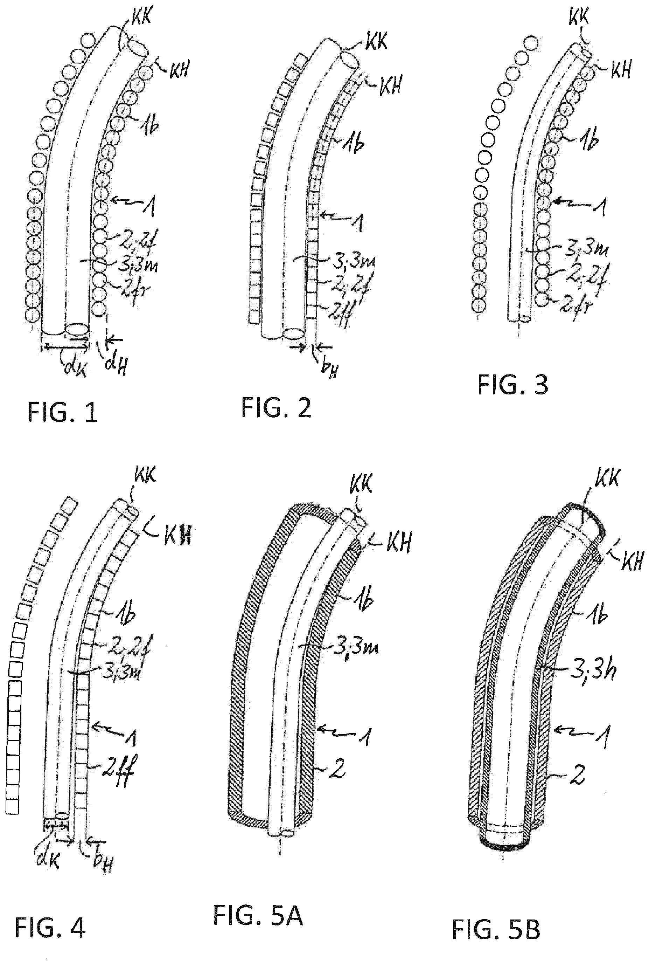

Wie in den Fig. 1 bis 42 in verschiedenen Ausführungen und Ansichten veranschaulicht, umfasst das erfindungsgemäße Funktionsschlauchinstrument einen schlauchartig langgestreckten Instrumentenkörper 1, der eine biegefähige Hülle 2 und einen sich in dieser erstreckenden, biegefähigen Kern 3 umfasst. Der Instrumentenkörper 1 ist dafür eingerichtet, eine axiale Relativbewegung von Hülle 2 und Kern 3 dergestalt zu ermöglichen, dass sich dadurch ein Axialhub AH mindestens in einem distalen Funktionsbereich 1a des Instrumentenkörpers zur Betätigung eines dort befindlichen Nutzelements ergibt. Des Weiteren beinhaltet das Funktionsschlauchinstrument eine Bedieneinheit 4, mit welcher der Instrumentenkörper 1 an einem dem distalen Funktionsbereich 1a proximal vorgelagerten Bedienbereich 1b zur Bewirkung des Axialhubs AH gekoppelt ist.

Die Bedieneinheit 4 ist dafür eingerichtet, bei Betätigung, insbesondere durch einen das Instrument verwendenden Benutzer, z.B. einen Arzt, den Instrumentenkörper 1 im Bedienbereich 1b reversibel von einem Ausgangspositionsverlauf VA in einen gegenüber diesem stärker gekrümmten Krümmungsverlauf VK zu biegen. Eine daraus resultierende Krümmungslängendifferenz von Hülle 2 und Kern 3 entlang des Krümmungsverlaufs VK stellt den Axialhub AH bereit. Vorzugsweise aber nicht notwendigerweise ist der Kern 3 in einem dem Bedienbereich 1b proximal vorgelagerten oder im Bedienbereich 1b selbst liegenden Fixierbereich 1c gegenüber der Hülle 2 axial bewegungsgesichert, d.h. er ist dort an der Hülle 2, z.B. durch Reibschluss oder eine Schweißverbindung oder dgl., oder zusammen mit dieser an einem dritten Bauteil dergestalt fixiert, dass er sich nicht oder jedenfalls nicht so stark axial relativ zur Hülle 2 bewegen kann, dass damit die Krümmungslängendifferenz ausgeglichen würde. Folglich überträgt sich die Krümmungslängendifferenz zwischen Kern 3 und Hülle 2 vorzugsweise im Wesentlichen ungemindert, alternativ teilweise, als Axialhub AH auf den distalen Funktionsbereich 1a.

Das Funktionsschlauchinstrument kann insbesondere zur Verwendung in der medizinischen Endoskopietechnik ausgelegt sein, d.h. als endoskopisches Funktionsschlauchinstrument. Wie sich für den Fachmann anhand der vorliegenden Erfindungsoffenbarung ergibt, kann das Instrument jedoch auch für anderweitige Zwecke eingesetzt werden, in denen Bedarf an einem schlauchförmig langgestreckten Instrument besteht, mit dem ein in einem distalen Bereich angeordnetes Nutzelement durch einen Axialhub zu betätigen ist, der vom Benutzer an der Bedieneinheit im proximalen Bedienbereich befehligt werden kann. Die gezeigten Ausführungsbeispiele eignen sich primär für entsprechende medizinische Führungsdrähte und Katheter mit distaler Nutzfunktion. In medizinischen Anwendungen des Instruments kann das Nutzelement beispielsweise ein entfaltbarer Drahtkorb oder Filter, z.B. zum Einfangen und/oder Entfernen von steinartigen Ablagerungen bzw. Gebilden wie Nierensteine oder von Blutgerinnseln zum Beseitigen bzw. Verhindern von Thrombosen, eine Schere, eine Zange, eine Kamera oder andere optische Komponente oder ein distaler Führungsdraht- oder Katheterabschnitt sein, der durch variables, gesteuertes Biegen bzw. Krümmen eine zugehörige Nutzfunktion erfüllt.

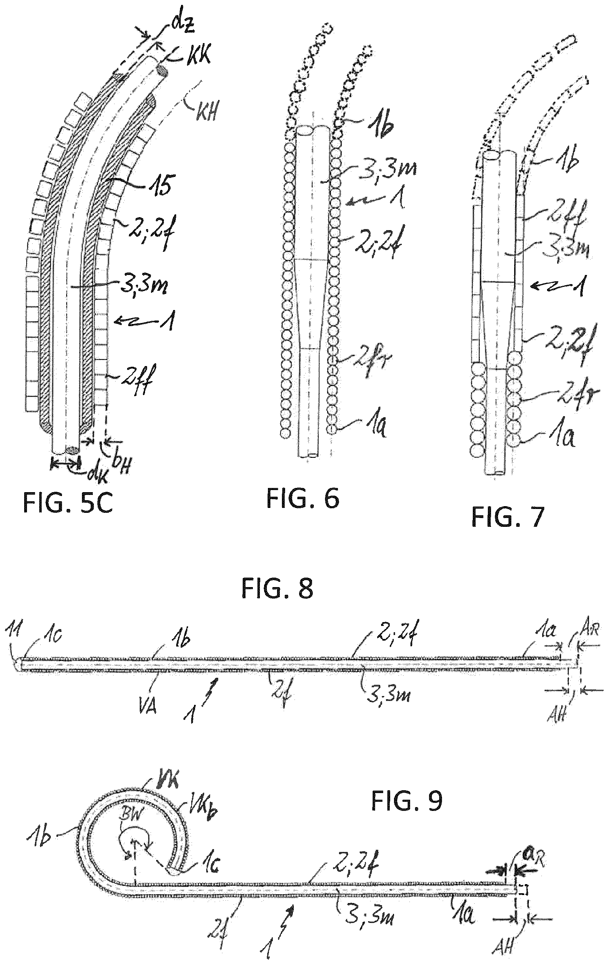

Die Bereitstellung des Axialhubs AH im distalen Funktionsbereich 1a durch Krümmen bzw. Biegen des Instrumentenkörpers 1 im Bedienbereich 1b ist schematisch und vergleichend in den Fig. 8 und 9 veranschaulicht. Fig. 8 zeigt den Instrumentenkörper 1 im Bedienbereich 1b mit seinem in diesem gewählten Beispiel geradlinigen Ausgangspositionsverlauf VA. Die Hülle 2 und der Kern 3 sind im Fixierbereich 1c, in diesem Fall dem proximalen Ende des Instrumentenkörpers 1, über eine fixierende proximale Endkappe 11 kraft- und/oder formschlüssig miteinander verbunden und dadurch in ihrer gegenseitigen axialen Lage bewegungsgesichert. Fig. 9 zeigt den Instrumentenkörper 1 mit seinem stärker gekrümmten Krümmungsverlauf VK im Bedienbereich 1b, wobei in diesem Beispiel der Krümmungsverlauf VK eine kreisbogenförmige Biegung des Instrumentenkörpers 1 im Bedienbereich 1b um einen Biegewinkel BW von ca. 300° beinhaltet. Daraus ergibt sich im distalen Funktionsbereich 1a der Axialhub AH, der in diesem Fall darin besteht, dass sich der Kern 3 um dieses Maß axial in der Hülle 2 zurückbewegt bzw. sich die Hülle 2 um dieses Maß relativ zum Kern 3 nach vorn bewegt. In den Fig. 8 und 9 ist dies dadurch weiter veranschaulicht, dass sich ein distales Ende des Kerns 3 im Ausgangspositionsverlauf VA des Instrumentenkörpers 1 gemäß Fig. 8 mit einem um den Axialhub AH größeren axialen Abstand A_{R} = a_{R} + AH vor einem distalen Ende der Hülle 2 befindet als dem Abstand a_{R}, wenn der Instrumentenkörper 1 gemäß Fig. 9 im Bedienbereich 1b den stärker gekrümmten Krümmungsverlauf VK einnimmt. Das Rückbewegen bzw. Rückverformen des Instrumentenkörpers 1 im Bedienbereich 1b vom stärker gekrümmten Krümmungsverlauf VK zum Ausgangspositionsverlauf VA hat entsprechend im distalen Funktionsbereich 1a die umgekehrte axiale Relativbewegung von Hülle 2 und Kern 3 um den Axialhub AH in entgegengesetzter Richtung zur Folge.

Der Axialhub AH im distalen Funktionsbereich 1a resultiert daraus, dass sich längs des Krümmungsverlaufs VKfür den Kern 3 eine größere Krümmungs- bzw. Weglänge ergibt als für die Hülle 2 und sich Hülle 2 und Kern 3 im proximal vorgelagerten Fixierbereich 1c nicht oder zumindest nicht in diesem Maß relativ zueinander axial bewegen können. Dabei ist angenommen, dass sich das Material der Hülle 2 ausgehend vom Ausgangspositionsverlauf VA an seiner radial inneren Seite im gekrümmten Verlauf VK nicht signifikant komprimieren lässt, jedoch dehnfähig ist und sich daher an seiner radial äußeren Seite im gekrümmten Verlauf VK dehnen kann. Dies gilt üblicherweise für alle praktisch verwendeten Hüllmaterialien von endoskopischen und vielen anderen Funktionsschlauchinstrumenten. Beispielsweise besteht die Hülle 2 bei endoskopischen Funktionsschlauchinstrumenten oftmals aus einem im Wesentlichen starren Metall- oder Kunststoffmaterial bzw. aus einem Schraubenfederkörper, bei dem die aufeinanderfolgenden Wicklungen im Ausgangspositionszustand, d.h. dem Ausgangspositionsverlauf VA, des Instrumentenkörpers 1 mit Berührkontakt gegeneinander anliegen.

In entsprechenden Ausführungen beinhaltet die Hülle 2 einen Schraubenfederkörper 2f aus einem Runddrahtmaterial 2fr, wie in den Ausführungsbeispielen der Fig. 1, 3, 6 und 8 bis 12. In anderweitigen Ausführungen beinhaltet die Hülle 2 einen Schraubenfederkörper 2f aus einem Flachdrahtmaterial 2ff, wie in den Fig. 2, 4, 5C und 13 veranschaulicht. Fig. 7 zeigt ein Ausführungsbeispiel, bei dem die Hülle 2 einen Schraubenfederkörper 2f beinhaltet, der im Bedienbereich 1b aus Flachdrahtmaterial 2ff gebildet ist, an den in einem distalen Bereich ein Abschnitt des Schraubenfederkörpers 2f aus Runddrahtmaterial 2fr anschließt. Alternativ ist die Hülle 2 aus einem flexiblen Vollwandmaterial gebildet, z.B. einem elastischen Metallrohrmaterial, insbesondere einem superelastischen Metalllegierungsrohrmaterial, oder einem Kunststoffschlauchmaterial. Die Fig. 5A und 5B zeigen derartige Ausführungsbeispiele. Diese eignen sich aufgrund der fluiddichten Vollwandausführung der Hülle 2 beispielsweise für Katheterinstrumente.

In entsprechenden Realisierungen beinhaltet der Kern 3 einen Massivdrahtkörper 3m, wie dies in den gezeigten Ausführungsbeispielen mit Ausnahme von Fig. 5B der Fall ist. In alternativen Realisierungen beinhaltet der Kern 3 einen Hohldrahtkörper 3h, wie dies beim Ausführungsbeispiel von Fig. 5B der Fall ist. Dabei kann der Kern 3 je nach Bedarf aus einem geeigneten draht- bzw. schlauchartigen Metall- und/oder Kunststoffmaterial bestehen. Es versteht sich, dass als weitere Varianten auch in den Ausführungen der Fig. 4 und 5A der dortige Massivdrahtkörper 3m durch einen Hohldrahtkörper nach Art des Hohldrahtkörpers 3h der Fig. 5B ersetzt sein kann. Der vom Hohldrahtkörper 3h gebildete Hohlkanal kann z.B. zum Durchführen von Medikamenten oder Kontrastmittel und anderen Fluiden oder von Nadeln und ähnlichen endoskopischen Funktionselementen genutzt werden.

Die Fig. 6, 7 und 13 veranschaulichen Beispiele, bei denen sich der Kern 3 bzw. Kerndraht in distaler Richtung über einen oder mehrere kegelstumpfförmige Abschnitte verjüngt. Dies kann in üblicher Weise dazu dienen, die Biegesteifigkeit des Instrumentenkörpers 1 in diesem Bereich, z.B. einem distalen Bereich, zu verringern. Außerdem kann mit dieser Maßnahme die Auswirkung von etwaigen Krümmungen des Instrumentenkörpers 1 in diesem Bereich auf die Bereitstellung des zur Nutzelementbetätigung gewünschten Axialhubs AH unterbunden bzw. abgeschwächt werden, wenn es im Gebrauch des Instruments zu derartigen Krümmungen in diesem Bereich kommt, beispielsweise aufgrund eines entsprechend gekrümmten Verlaufs eines Gewebekanals, in den der Instrumentenkörper 1 eingeführt wird.

Zur näheren Erläuterung des vorliegend genutzten Effekts der Axialhubbereitstellung im distalen Funktionsbereich 1a durch die Krümmungsänderung im Bedienbereich 1b des Instrumentenkörpers 1 wird auf die exemplarischen Fig. 1 bis 5C Bezug genommen. Es sei an dieser Stelle angemerkt, dass die Darstellung des Instruments und seiner Komponenten in den Fig. 1 bis 33 überwiegend nicht maßstäblich ist, sondern einzelne Bestandteile je nach Bedarf gegenüber anderen vergrößert wiedergegeben sind, was dem leichteren Verständnis dient.

In den Fig. 1 bis 5C ist der Instrumentenkörper 1 jeweils mit einem geradlinigen unteren Teil und einem sich demgegenüber krümmenden oberen Teil gezeigt. Im Fall des Runddrahtmaterials 2fr gemäß den Fig. 1 und 3 bleiben die aufeinanderfolgenden Wicklungen an der radial inneren Seite des Krümmungsbogens in gegenseitigem Berührkontakt, wodurch die Krümmungslänge der Hülle 2 bestimmt ist, während sich an der radial äußeren Krümmungsseite die Wicklungen zum Dehnen der Hülle 2 voneinander entfernen. Bei Umbiegung in einen Vollkreis entspricht folglich die Umfangslänge einer zugehörigen Krümmungsbahn KH der radial inneren Krümmungsseite der Hülle 2 unverändert der Axialerstreckung der Hülle 2 im nicht gekrümmten, geradlinigen Ausgangszustand, da dort die inkompressiblen Federwicklungen in Berührkontakt bleiben, während sich eine demgegenüber größere Umfangslänge für eine Krümmungsbahn KK des Kerns 3 ergibt, da der Krümmungsradius der Krümmungsbahn KK des Kerns 3 um die Hälfte eines Durchmessers d_{K} des Kerns 3 zuzüglich der Hälfte eines Durchmessers bzw. Wandstärke d_{H} des Runddrahtmaterials 2fr der Hülle 2 größer ist als der Krümmungsradius der inneren Krümmungsbahn KH der Hülle 2. Dies entspricht einer axialen Längendifferenz D_{L} zwischen Kern 3 einerseits und Hülle 2 andererseits beim Strecken einer solchen Vollkreiswicklung in den axial geradlinigen Verlauf in Höhe von D_{L} = *π*(d_{K}+d_{H})/2. Durch das Krümmen des Instrumentenkörpers 1 von einem geradlinigen Verlauf in einen Vollkreis und beim Strecken aus dem Vollkreis in den geradlinigen Verlauf stellt diese Krümmungslängendifferenz D_{L} den Axialhub AH dar, um den sich Kern 3 und Hülle 2 in einem distal davor liegenden Bereich axial relativ bewegen, wenn sie im gegenüberliegenden proximalen Bereich axial bewegungsgesichert sind. Wenn der Instrumentenkörper 1 nur auf einem Kreisbogen mit einer Umfangswinkelerstreckung kleiner als 360° gebogen wird, resultiert dies entsprechend in einem Bruchteil des Wertes der Längendifferenz D_{L} für den Vollkreis als Axialhub AH, d.h. AH=D_{L}×α_{B}/360°, mit α_{B} als Kreisbogenwinkel.

Wie aus diesem Beispielfall deutlich wird, tritt der Axialhub AH prinzipbedingt durch die Krümmung des dergestalt gebauten Instrumentenkörpers 1 auf, wobei der Wert des Axialhubs AH, d.h. die zugehörige Hublänge, von den konkreten Gegebenheiten abhängig ist, insbesondere von der Dimensionierung des Kerns 3 und der Hülle 2 und davon, ob bzw. in welchem Maß die beteiligten Materialien komprimierbar sind. Es versteht sich, dass für diesen Effekt und damit für die Bereitstellung des Axialhubs AH nicht zwingend erforderlich ist, dass der Instrumentenkörper 1 längs einer Kreisbahn gebogen wird. Vielmehr ist alternativ jeder andere Biegeverlauf geeignet, der zu der erforderlichen Krümmungslängendifferenz führt.

Für das Beispiel von Fig. 2 gelten die analogen Betrachtungen wie für den Fall von Fig. 1, wobei sich in diesem Fall für die Längendifferenz D_{L} die Beziehung D_{L} = *π*(d_{K}/2+b_{H}) ergibt, da sich in diesem Fall die Federwicklungen des Flachdrahtmaterials 2ff mit Wandbreite b_{H} an ihrer radial inneren Seitenkante gegeneinander anlegen, wenn die Hülle 2 gekrümmt wird. Für die Beispiele der Fig. 3 und 4 gelten die obigen Betrachtungen zu den Beispielen der Fig. 1 und 2 analog. Zwar ist in diesen Fällen der Innendurchmesser der Hülle 2 deutlich größer als der Außendurchmesser des Kerns 3, während bei den Beispielen der Fig. 1 und 2 der Innendurchmesser der Hülle 2 nur so viel größer als der Außendurchmesser des Kerns 3 ist, dass die gewünschte axiale Relativbewegung ermöglicht ist, beim Biegen des Instrumentenkörpers 1 legt sich jedoch der Kern 3 radial innen gegen die Innenseite der Hülle 2 an, da der Instrumentenkörper 1 durch das Krümmen unter Zugspannung gesetzt wird.

Des Weiteren gelten die obigen Betrachtungen anhand der Fig. 1 und 2 analog für das Beispiel von Fig. 5A. Auch bei diesem Beispiel tritt aus dem gleichen Grund der Axialhub AH durch Veränderung des Krümmungsverlaufs des Instrumentenkörpers 1 auf, wobei lediglich der konkrete Wert der Hublänge vom eventuell gegenüber dem Schraubenfederkörper 2f anderen Material der Hülle 2 abhängen kann, insbesondere von dessen Kompressions- und/oder Dehnungsverhalten. Dies lässt sich bei Bedarf z.B. experimentell bestimmen. Ebenso gelten die obigen Betrachtungen analog auch für Realisierungen, bei denen der Kern 3 aus einem Hohldrahtkörper gebildet ist, wie dem Hohldrahtkörper 3h im Fall von Fig. 5B.

Aus der obigen Betrachtung ergibt sich weiter, dass der Axialhub AH im Wesentlichen von der Krümmungslängendifferenz der beiden Krümmungsbahnen KK und KH von Kern 3 einerseits und Hülle 2 andererseits abhängig ist. In Ausführungen, bei denen der Außendurchmesser des Kerns 3 deutlich kleiner ist als der Innendurchmesser der Hülle 2, kann daher der Axialhub AH bei gleichem Krümmungswinkel dadurch erhöht werden, dass zwischen Kern 3 und Hülle 2 eine Abstandshülse 15 vorgesehen ist, die den Kern 3 um das Maß ihrer Wanddicke d_{z} radial gegenüber der Hülle 2 auf Abstand hält, wie in Fig. 5C für eine entsprechende Variante des Beispiels von Fig. 4 gezeigt. Verglichen mit dem Beispiel von Fig. 4 trägt in diesem Fall die Wanddicke d_{z} der Abstandshülse 15 zusätzlich zur Krümmungslängendifferenz der beiden Krümmungsbahnen KK und KH von Kern 3 und Hülle 2 und damit zum daraus resultierenden Axialhub AH bei.

In typischen medizinischen Endoskopie-Anwendungen, wie für Führungsdrähte und Katheter, liegt der Durchmesser der Hülle 2 meist bei wenigen Zehntel Millimetern bis wenigen Millimetern und somit die Längendifferenz D_{L} für die Vollkreiswicklung und damit der zugehörig bewirkte Axialhub AH meist in der Größenordnung von ca. einem Zehntel Millimeter bis ein oder zwei bzw. einige Millimeter. Folglich lassen sich mit einer relativ großen und damit gut handhabbaren Biegebewegung des Instrumentenkörpers 1 auch sehr geringe Hublängen für den Axialhub AH bequem einstellen, indem der Instrumentenkörper 1 nur längs eines bogenförmigen Abschnitts um weniger als 360° gebogen wird. Zur Erzielung größerer Hublängen für den Axialhub A kann der Instrumentenkörper 1 um entsprechend mehr als 360° gebogen werden. Dies schließt insbesondere die Möglichkeit ein, den Instrumentenkörper 1 in eine Mehrzahl von Vollkreiswicklungen VK_{w} bzw. mehrmals um 360° zu biegen und dadurch die Hublänge, die sich mit einer einzelnen Vollkreiswicklung VK_{w} ergibt, entsprechend zu vervielfachen.

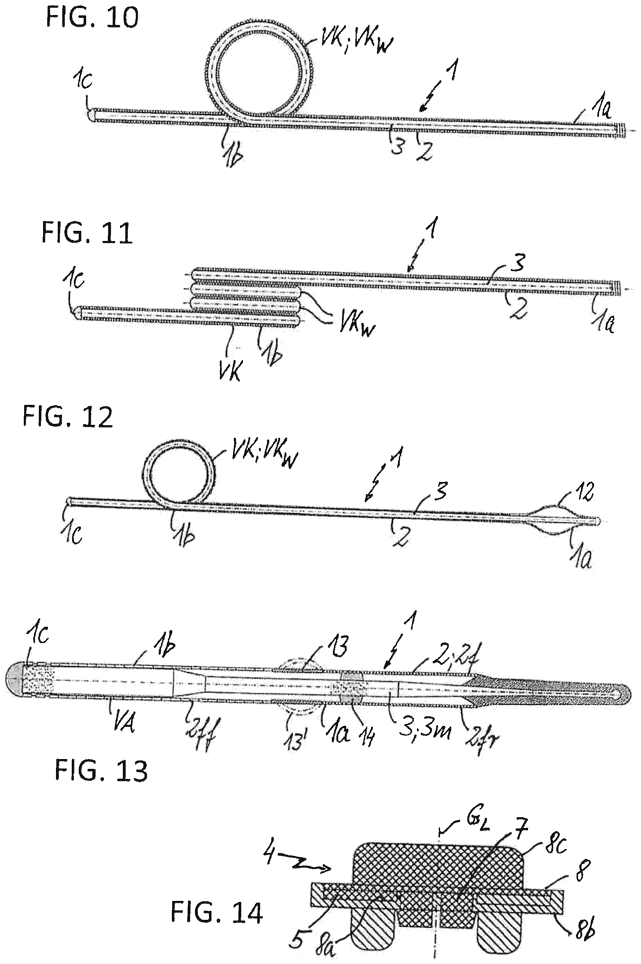

Fig. 12 veranschaulicht die Nutzung des Axialhubs AH zur Betätigung eines Nutzelements in Form eines auffaltbaren Drahtkorbs 12 im distalen Funktionsbereich 1a des Instrumentenkörpers 1, in diesem Fall speziell in einem distalen Endbereich des Instruments. Fig. 12 zeigt das Instrument mit dem Instrumentenkörper 1 im gegenüber dem geradlinigen Ausgangspositionsverlauf VA stärker gekrümmten, einen oder mehrere Vollkreiswicklungen VK_{w} umfassenden Krümmungsverlauf VK, wodurch sich das distale Ende des Kerns 3 relativ zum distalen Ende der Hülle 2 zurückbewegt, wie oben zu den Fig. 8 und 9 erläutert. Dies staucht den an der Hülle 2 ausgebildeten Drahtkorb 12, der sich dadurch radial in die gezeigte Position aufweitet.

Fig. 13 zeigt ein Ausführungsbeispiel, bei dem als durch den Axialhub AH betätigtes Nutzelement ein auffaltbarer Drahtkorb 13 an der Hülle 2 an einer Position im distalen Funktionsbereich 1a proximal hinter einem distalen Endbereich ausgebildet ist. Zur Verringerung der Biegesteifigkeit ist der Kern 3 in Richtung distalem Endbereich verjüngt, wobei in distaler Richtung hinter dem Drahtkorb 13 eine stabilisierende Fixierung 14 von Kern 3 und Hülle 2 vorgesehen ist. Durch Krümmen des Instrumentenkörpers 1 im Bedienbereich 1b aus dem gezeigten, geradlinigen Ausgangspositionsverlauf VA heraus kommt es wiederum zu einer axialen Rückbewegung des Kerns 3 relativ zur Hülle 2, wodurch der Kern 3 die Hülle 2 über die Fixierung 14 mit einer stauchenden Schubkraft in proximaler Richtung beaufschlagt, durch die sich der Drahtkorb 13 in seine gestrichelt dargestellte Funktionsposition 13' radial aufweitet.

In entsprechenden Realisierungen beinhaltet der Krümmungsverlauf VK des Instrumentenkörpers 1 im Bedienbereich 1b einen oder mehrere bogenförmige Abschnitte VK_{b}, wie beispielsweise in den Fig. 9, 17, 18, 20 bis 22, 24 bis 26, 29 bis 32, 35, 36 und 42 ersichtlich.

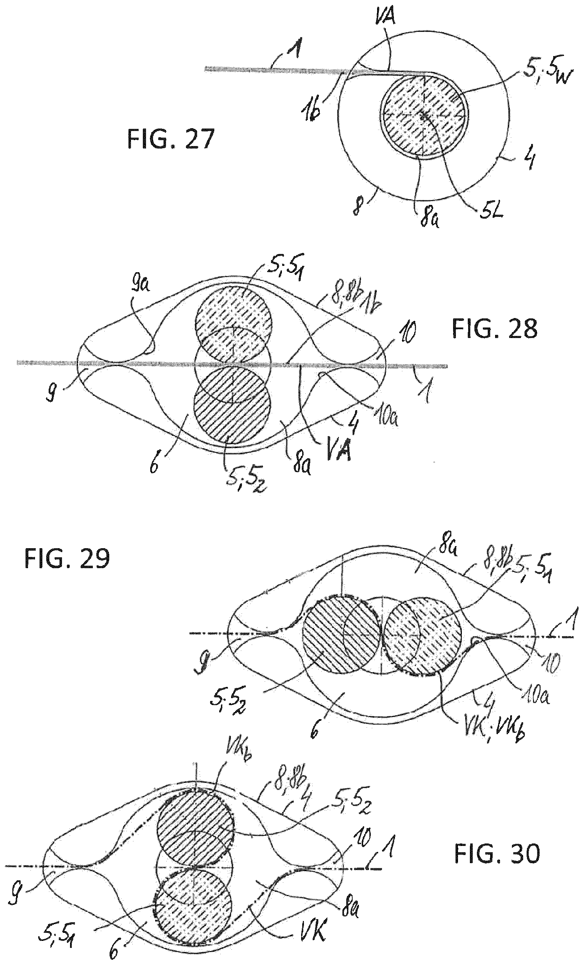

In entsprechenden Realisierungen umfasst der Krümmungsverlauf VK des Instrumentenkörpers 1 im Bedienbereich 1b einen oder mehrere Vollwicklungen VK_{w}, wie dies in den Ausführungsbeispielen der Fig. 10 bis 12 und ggf. auch in Fig. 27 der Fall ist.

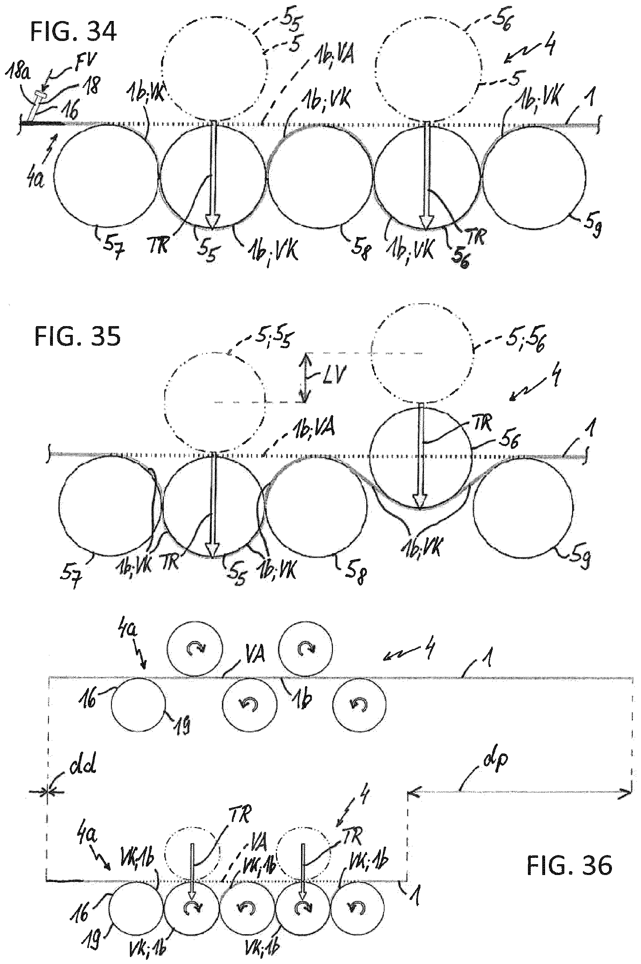

In entsprechenden Ausführungen weist die Bedieneinheit 4 in einem distalen Endbereich 4a eine die Hülle 2 gegen Axialbewegung sichernde Hüllenfixierung 16 auf, wie in den Fig. 34 und 36 bis 40 gezeigt. Dieses axiale Festlegen der Hülle 2 vermeidet, dass das Krümmen des Instrumentenkörpers 1 im Bedienbereich 1b eine entsprechende Veränderung der axialen Position der Hülle 2 im distalen Funktionsbereich 1a verursacht, und erleichtert folglich die Positionierung des dort angeordneten Nutzelementes am gedachten Einsatzort. Die für das Krümmen benötigte Zusatzlänge des Instrumentenkörpers 1 im Bedienbereich 1b wird in diesem Fall vom proximal vorgelagerten Teil des Instrumentenkörpers 1 nachgeliefert. Dies gilt sowohl für die Hülle 2 als auch für den Kern 3, wenn dieser, wie oben erwähnt, im Fixierbereich 1c gegenüber der Hülle 2 axial bewegungsgesichert fixiert ist. Wie gewünscht, überträgt sich dann nur die durch das Krümmen des Instrumentenkörpers 1 im Bedienbereich 1b vom Ausgangspositionsverlauf VA in den Krümmungsverlauf VK zusätzlich bewirkte Krümmungslängendifferenz zwischen Kern 3 und Hülle 2 als Axialhub AH auf den distalen Funktionsbereich 1a des Instrumentenkörpers 1.

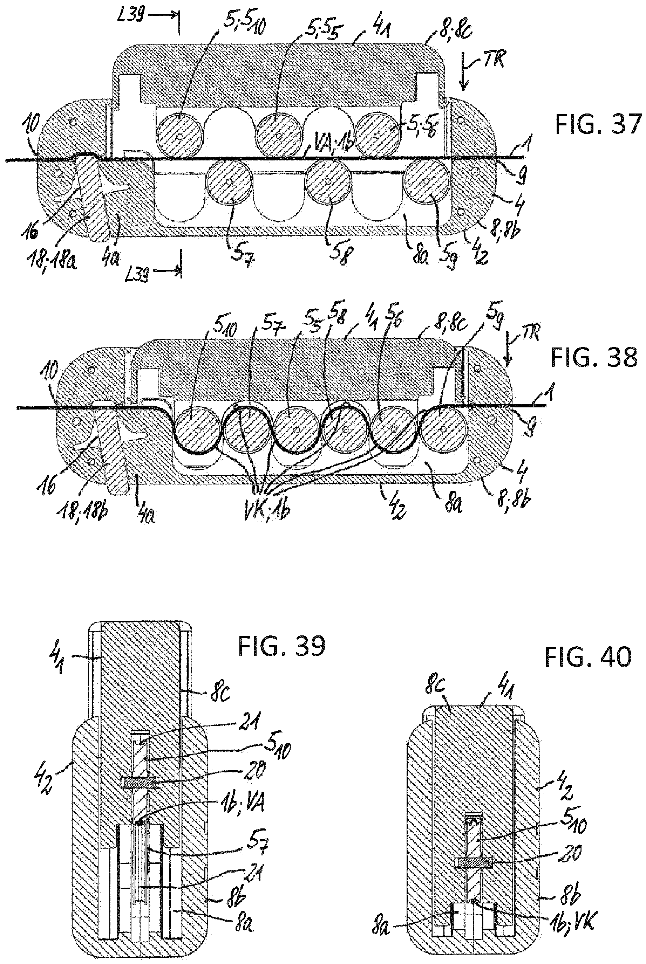

In den Ausführungsbeispielen der Fig. 34 und 37 bis 40 beinhaltet die Hüllenfixierung 16 einen Klemmstift 18, der zwischen einer Klemmposition 18a, siehe die Fig. 34 und 37, und einer Löseposition 18b, siehe Fig. 38, bewegbar ist. In der Klemmposition 18a klemmt er die Hülle 2 axial fest, z.B. an einem Gehäuseteil der Bedieneinheit 4, in der Löseposition 18b gibt er die Hülle 2 frei, wodurch der Instrumentenkörper axial bewegt werden kann, z.B. um ihn in die Bedieneinheit 4 einzufügen oder ihn aus dieser herauszunehmen.

Im Ausführungsbeispiel der Fig. 36 beinhaltet die Hüllenfixierung 16 einen unbewegt z.B. an einem Gehäuseteil der Bedieneinheit 4 gehaltenen Biegekörper 19, gegen den sich die Hülle 2 beim Krümmen durch die Betätigung der Bedieneinheit 4 mit gegen Entlanggleiten sicherndem Reibschluss anlegt, wodurch die Hülle 2 dort in ihrer axialen Lage festgehalten wird. Dieser Biegekörper 19 kann z.B., wie gezeigt, ein drehfest gehaltener Zylinderkörper sein, gegen dessen Mantelfläche die Hülle 2 reibschlüssig zur Anlage kommt.

In vorteilhaften Ausführungsformen weist die Bedieneinheit 4 mindestens einen bewegten Biegekörper 5 auf, gegen dessen Umfang sich der Instrumentenkörper 1 in seinem Krümmungsverlauf VK anlegt. Dies ist insbesondere anhand der Fig. 14 bis 42 ersichtlich. In den gezeigten Beispielen ist der bewegte Biegekörper 5 als Zylinderkörper ausgebildet, alternativ kann er je nach Bedarf eine andere Form haben, z.B. eine polygonale Form, eine ovale bzw. elliptische Form oder eine Kugelform. Es versteht sich, dass in entsprechenden Ausführungsvarianten je nach Bedarf entweder nur ein bewegter Biegekörper oder zwei bewegte Biegekörper oder mehr als zwei bewegte Biegekörper vorhanden sein können.

In entsprechenden Realisierungen ist der bewegte Biegekörper 5 um seine Längsachse 5L drehbeweglich und dient dazu, den Instrumentenkörper 1 im Bedienbereich 1b aufzuwickeln und ihn auf diese Weise dort in seinen Krümmungsverlauf VK zu verbringen. Fig. 27 zeigt beispielhaft die Bedieneinheit 4 in einer solchen Ausführung. Der bewegte Biegekörper 5 bildet in diesem Fall einen Wickelkörper 5_{w}, an dessen Umfang der Instrumentenkörper 1 mit einem proximalen Ende fixiert ist, zweckmäßigerweise durch eine zugehörige form- und/oder kraftschlüssige Verbindung. Durch entsprechendes Drehen des Wickelkörpers 5_{w} wird der Instrumentenkörper 1 in seinem Bedienbereich 1b auf diesen aufgewickelt bzw. wieder von diesem abgewickelt. Der zugehörige Krümmungsverlauf VK kann dementsprechend einen oder mehrere Vollwicklungen um den Wickelkörper 5_{w} herum oder nur einen bogenförmigen Abschnitt mit einer Umfangswinkelerstreckung kleiner als 360° beinhalten.

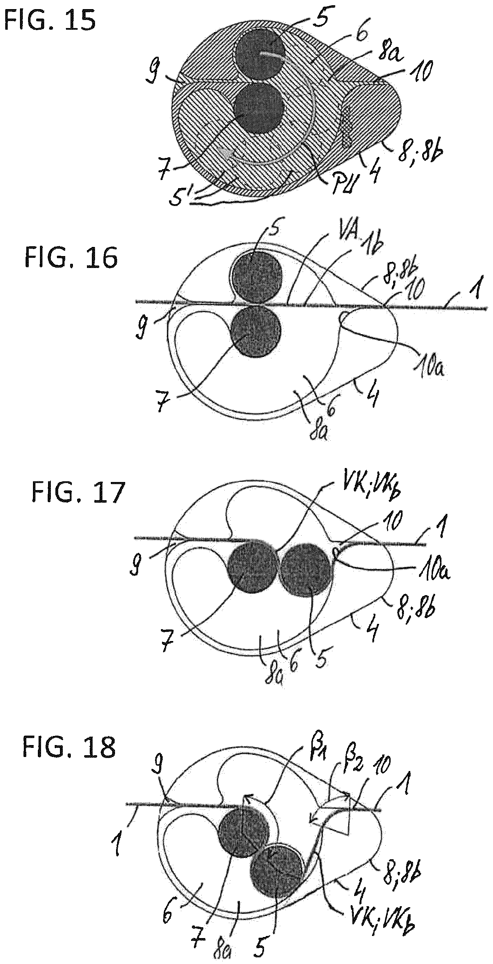

In anderweitigen Ausführungsformen läuft der bewegte Biegekörper 5 auf einer Umlaufbahn 6 um und nimmt den Instrumentenkörper 1 zum Verbringen in seinen Krümmungsverlauf VK bei dieser Umlaufbewegung mit. Die Fig. 15 bis 26 und 28 bis 33 veranschaulichen zugehörige Beispiele.

In entsprechenden Ausführungen erstreckt sich die Umlaufbahn 6 um einen zentrischen Biegekörper 7 herum, wobei sich der Instrumentenkörper 1 im Bedienbereich 1b in seinem Krümmungsverlauf VK gegen den Umfang des zentrischen Biegekörpers 7 anlegt. Zugehörige Ausführungsbeispiele sind in den Fig. 14 bis 26 veranschaulicht. Der zentrische Biegekörper 7 kann je nach Bedarf um seine Längsachse drehbeweglich oder alternativ unbeweglich angeordnet sein.

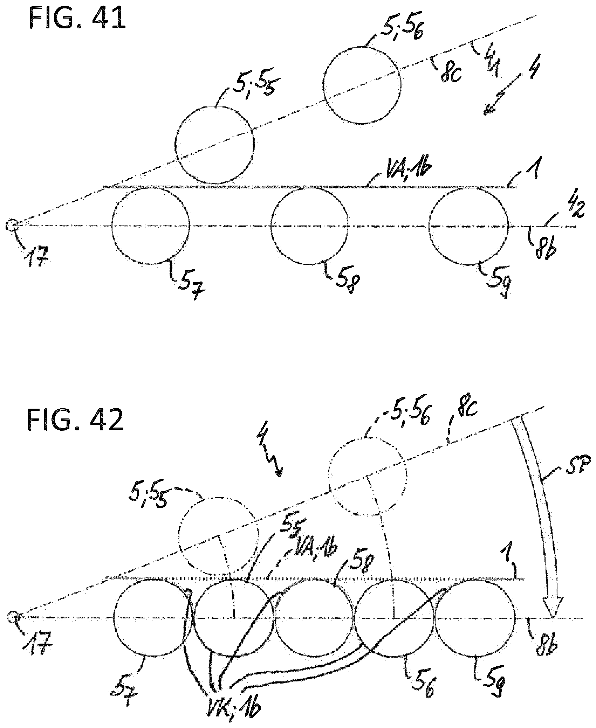

In weiteren Ausführungsformen ist der bewegte Biegekörper 5 um eine Schwenkachse 17 verschwenkbar und nimmt den Instrumentenkörper 1 zum Verbringen in seinen Krümmungsverlauf VK mit. Die Fig. 41 und 42 zeigen ein zugehöriges Ausführungsbeispiel, wobei Fig. 41 die Ausgangsposition der Bedieneinheit 4 zeigt und Fig. 42 das Verbringen des Instrumentenkörpers 1 von dieser Ausgangsposition in seinen Krümmungsverlauf VK veranschaulicht.

In weiteren vorteilhaften Ausführungen ist der bewegte Biegekörper 5 längs einer Verschiebungsrichtung TR translationsbeweglich und nimmt den Instrumentenkörper 1 zum Verbringen in seinen Krümmungsverlauf VK mit. Die Fig. 34 bis 40 zeigen zugehörige Ausführungsbeispiele.

Es versteht sich, dass in weiteren, nicht gezeigten Ausführungsformen mehrere der genannten Bewegungsmöglichkeiten für den mindestens einen bewegten Biegekörper 5 kombiniert sein können. Beispielsweise kann der bewegte Biegekörper 5 kombiniert dreh- und translationsbeweglich oder kombiniert schwenk- und translationsbeweglich oder kombiniert dreh- und umlaufbeweglich etc. angeordnet sein.

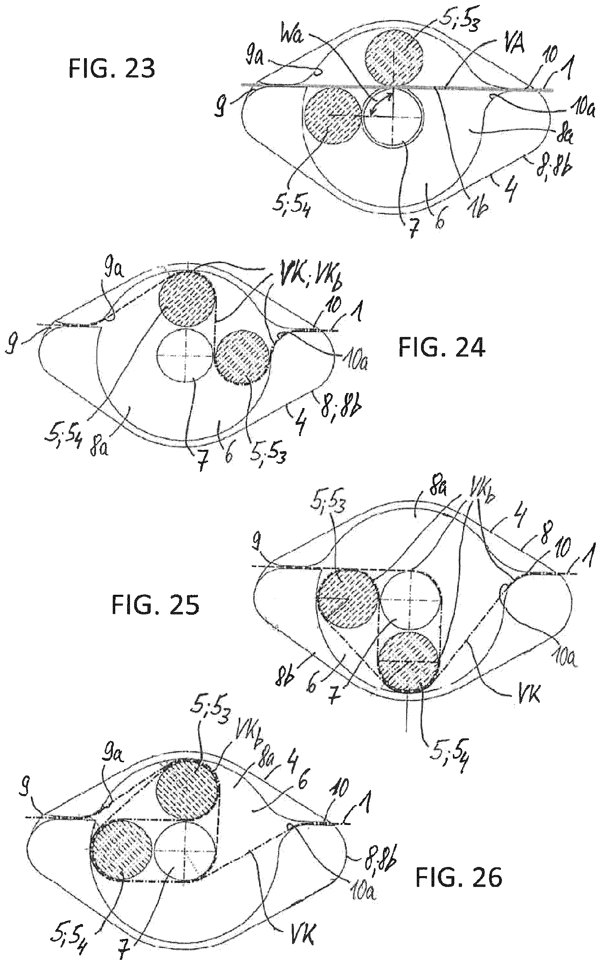

In entsprechenden Ausführungsformen weist die Bedieneinheit 4 einen ersten und einen zweiten bewegten Biegekörper 5₁, 5₂ bzw. 5s, 5₄ auf, gegen deren Umfang sich der Instrumentenkörper 1 in seinem Krümmungsverlauf VK anlegt, wobei diese Biegekörper 5₁, 5₂; 5s, 5₄ mit umlaufseitigem Abstand, d.h. Umfangswinkelabstand, auf der Umlaufbahn 6 umlaufen und den Instrumentenkörper 1 im Bedienbereich 1b zum Verbringen in seinen Krümmungsverlauf VK mitnehmen. Die Fig. 26 und 28 bis 32 veranschaulichen zugehörige Ausführungsbeispiele.

In vorteilhaften Ausführungen liegen sich der erste und der zweite Biegekörper 5₁, 5₂ diametral auf der Umlaufbahn 6 gegenüber und führen zwischen sich den Instrumentenkörper 1. Die Fig. 28 bis 33 veranschaulichen zugehörige Beispiele. Bei diesen gezeigten Ausführungsbeispielen entfällt der zentrische Biegekörper 7, und die beiden auf der Umlaufbahn 6 umlaufenden Biegekörper 5₁, 5₂ führen zwischen sich den Instrumentenkörper 1 in seinem geradlinigen Ausgangspositionsverlauf VA mit Berührkontakt bzw. engem Abstand.

In entsprechenden Realisierungen sind der erste und der zweite Biegekörper 5s, 5₄ um einen Umlaufbahnwinkel Wa kleiner als 180° voneinander beabstandet und laufen um den zentrischen Biegekörper 7 um. Die Fig. 23 bis 26 zeigen ein zugehöriges Beispiel. In diesem gezeigten Ausführungsbeispiel beträgt der beabstandende Umlaufbahnwinkel Wa ca. 90°. Je nach Bedarf kann der Umlaufbahnwinkel Wa einen anderen Wert haben, z.B. zwischen 80° und 90° oder zwischen 90° und 100°.

In vorteilhaften Ausführungsformen weist die Bedieneinheit 4 ein Bediengehäuse 8 mit einem Gehäuseinnenraum 8a auf, in dem sich die Umlaufbahn 6 befindet und das den mindestens einen bewegten Biegekörper 5 sowie den Bedienbereich 1b des Instrumentenkörpers 1 aufnimmt. Dabei beinhaltet das Bediengehäuse 8 für den Instrumentenkörper 1 eine Eintrittsöffnung 9 in den Gehäuseinnenraum 8a und eine Austrittsöffnung 10 aus dem Gehäuseinnenraum 8a. Die Fig. 14 bis 26, 28 bis 33 und 37 bis 40 veranschaulichen verschiedene Varianten der dergestalt konfigurierten Bedieneinheit 4. Wie daraus ersichtlich, kann das Bediengehäuse 8 z.B. eine relativ flache, scheibenartige Form besitzen, d.h. es genügt schon eine relativ geringe Höhenausdehnung des Bediengehäuses 8 in der Richtung senkrecht zur Umlaufbahn 6 bzw. parallel zu einer in Fig. 14 markierten Längsachse G_{L} des Bediengehäuses 8. Die Ausführung gemäß den Fig. 28 bis 33 ermöglicht durch den Wegfall des zentrischen Biegekörpers 7 bei Bedarf eine vergleichsweise kompakte Bauform für die Bedieneinheit 4 bzw. das Bediengehäuse 8 auch in der Querebene parallel zur Ebene der Umlaufbahn 6. Auch im Ausführungsbeispiel der Fig. 27 beinhaltet die Bedieneinheit 4 das Bediengehäuse 8 mit Gehäuseinnenraum 8a, wobei in diesem Fall der als Wickelkörper 5_{w} fungierende Biegekörper 5 im Gehäuseinnenraum 8a aufgenommen ist. Bei der Ausführung gemäß den Fig. 37 bis 40 sind im Gehäuseinnenraum 8a zwei Gruppen von jeweils mehreren, vorzugsweise zylindrischen, Biegekörpern 5 aufgenommen, die relativ zueinander quer zum geradlinigen Ausgangspositionsverlauf VA des Instrumentenkörpers 1 von der Eintrittsöffnung 9 zur Austrittsöffnung 10 translationsbeweglich sind.

In vorteilhaften Ausführungen umfasst das Bediengehäuse 8 zwei relativ zueinander drehbewegliche oder schwenkbewegliche oder translationsbewegliche Gehäuseteile 8b, 8c, wobei die Eintrittsöffnung 9 und die Austrittsöffnung 10 am einen Gehäuseteil 8b und der mindestens eine bewegte Biegekörper 5 am anderen Gehäuseteil 8c angeordnet sind. In dieser Ausführung kann der Benutzer die Bedieneinheit 4 sehr bequem dadurch betätigen, dass er die beiden Gehäuseteile 8b, 8c entsprechend relativ zueinander bewegt, d.h. verdreht oder verschwenkt oder verschiebt, wodurch sich der oder die betreffenden Biegekörper 5 entlang der Umlaufbahn 6 bewegen und den Instrumentenkörper 1 in seinem im Gehäuseinnenraum 8a befindlichen Bedienbereich 1b in den stärker gekrümmten Krümmungsverlauf VK biegen bzw. verformen. Durch die umgekehrte relative Bewegung lässt sich der Instrumentenkörper 1 wieder in seinen Ausgangspositionsverlauf AV zurückverformen bzw. erreicht diesen Zustand selbsttätig nach Biegeentlastung durch den oder die Biegekörper 5. Die Fig. 14 bis 26 und 28 bis 33 stellen entsprechende Ausführungsbeispiele mit relativ zueinander drehbeweglichen Gehäuseteilen 8b, 8c dar. Die Fig. 37 bis 40 veranschaulichen ein Ausführungsbeispiel, bei dem die beiden Gehäuseteile 8b, 8c relativ zueinander translationsbeweglich sind. Analog kann das in den Fig. 41 und 42 schematisch gezeigte Ausführungsbeispiel in einer praktischen Umsetzung die beiden Gehäuseteile 8b, 8c in einer zueinander scherenartig schwenkbeweglichen Konfiguration umfassen.

In vorteilhaften Ausführungen sind die beiden Gehäuseteile 8b, 8c, wie in den gezeigten Beispielen, mittels einer lösbaren Schnapp- bzw. Clips- oder Rastverbindung drehbeweglich aneinander gehalten. Der Benutzer kann in diesem Fall den Instrumentenkörper 1 z.B. in einen der noch nicht aneinander montierten Gehäuseteile 8b, 8c einlegen und anschließend den anderen Gehäuseteil aufschnappen bzw. aufclipsen oder aufrasten. Durch Lösen der beiden Gehäuseteile 8b, 8c kann er bei Bedarf den Instrumentenkörper 1 entnehmen bzw. austauschen.

Bei den beiden Gehäuseteilen 8b, 8c kann es sich insbesondere um zwei Gehäusehälften handeln, die zusammen ein Außengehäuse der Bedieneinheit 4 bilden, wie dies in den gezeigten Beispielen der Fall ist.

Die Fig. 15 bis 42 veranschaulichen einige exemplarische Beispiele zur Bereitstellung des Axialhubs AH durch stufenloses Verändern der Krümmungslänge des Instrumentenkörpers 1 mittels der Bedieneinheit 4 in unterschiedlichen Varianten der Bedieneinheit 4.

Im Ausführungsbeispiel der Fig. 15 bis 18 ist die Umlaufbahn 6 für den umlaufenden Biegekörper 5 um den zentrischen Biegekörper 7 herum auf ca. 270° begrenzt. Fig. 15 zeigt die Bedieneinheit 4 in einem Ausgangszustand noch ohne den Instrumentenkörper 1, wobei die Umlaufbewegung des Biegekörpers 5 durch einen Umlaufpfeil PU symbolisiert ist und exemplarisch einige Zwischenpositionen 5' des Biegekörpers auf der Umlaufbahn 6 gestrichelt angegeben sind. Fig. 16 zeigt die Bedieneinheit 4 mit zusätzlich eingelegtem bzw. durchgeführtem Bedienbereich 1b des Instrumentenkörpers 1.

Fig. 17 veranschaulicht die Situation nach einem Herausdrehen des umlaufenden Biegekörpers 5 aus seiner Anfangsposition um ca. 90° auf der Umlaufbahn 6. Dies wird vom Benutzer durch entsprechendes relatives Verdrehen der Gehäuseschalen 8b, 8c an der Bedieneinheit 4 bewirkt. In dieser Gebrauchssituation hat der umlaufende Biegekörper 5 den Instrumentenkörper 1 so weit mitgenommen, dass sich der Instrumentenkörper 1 in einem Viertelkreis gegen den zentrischen Biegekörper 7 anlegt und in einem Halbkreis gegen den umlaufenden Biegekörper 5. Zusätzlich verursacht eine Austrittsschräge 10a der Austrittsöffnung 10 des Bediengehäuses 8 eine weitere Krümmung des Instrumentenkörpers 1 um ca. 90°, so dass sich insgesamt ein Gesamtkrümmungswinkel des Instrumentenkörpers 1 von ca. 360° ergibt.

Fig. 18 zeigt die Situation, wenn der umlaufende Biegekörper 5 um einen Winkel β₁ von ca. 135° aus seinem Anfangszustand heraus auf der Umlaufbahn 6 gedreht wurde. In dieser Lage legt sich der Instrumentenkörper 1 entsprechend um einen Winkel von 135° gegen den zentrischen Biegekörper 7 und anschließend um einen Umfangswinkel von ca. 206° gegen den umlaufenden Biegekörper 5 an. Die Austrittsschräge 10a bewirkt eine weitere Biegung des Instrumentenkörpers 1 um einen Winkel β₂ von ca. 71°. Insgesamt resultiert dies in einem Gesamtkrümmungswinkel von ca. 412°.

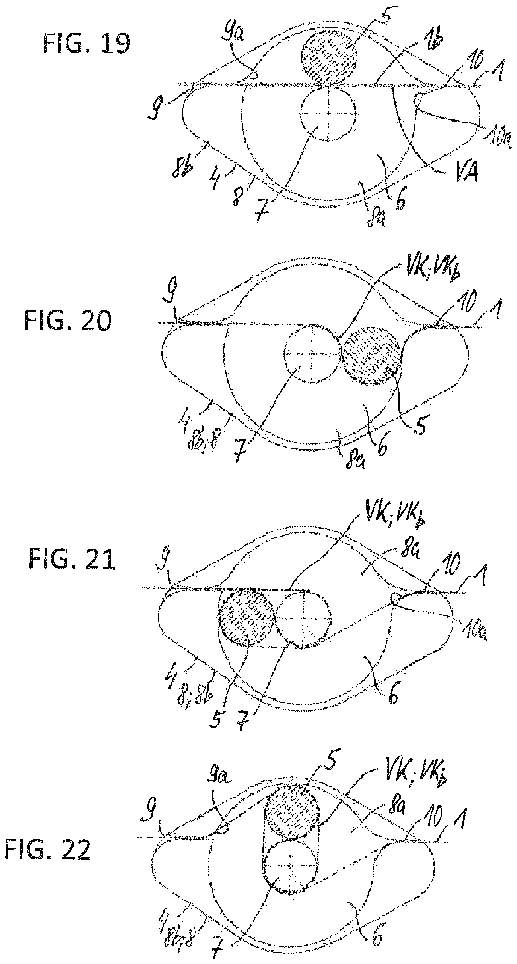

Das Ausführungsbeispiel der Fig. 19 bis 22 ähnelt demjenigen der Fig. 15 bis 18 mit der Ausnahme, dass die Umlaufbahn 6 nicht beschränkt ist, sondern vollständig um den zentrischen Biegekörper 7 umläuft. Entsprechend ist die Umlaufbewegung des umlaufenden Biegekörpers 5 auf der Umlaufbahn 6 nicht beschränkt.

Fig. 19 zeigt die Anordnung im Ausgangszustand mit im Bedienbereich 1b geradlinig durch das Bediengehäuse 8 der Bedieneinheit 4 durchgeführtem Instrumentenkörper 1. Fig. 20 zeigt die Situation mit um 90° auf der Umlaufbahn 6 bewegtem Biegekörper 5, woraus der gleiche Gesamtkrümmungswinkel des Instrumentenkörpers 1 resultiert wie in oben erläuterten, analogen Situation von Fig. 17 mit einer Gesamtbiegung des Instrumentenkörpers 1 von ca. 360°.

Fig. 21 zeigt die Anordnung mit um 270° aus dem Anfangszustand heraus auf der Umlaufbahn 6 bewegtem Biegekörper 5. In diesem Fall hat sich der Instrumentenkörper 1 in einem Umfangswinkel von ca. 270° gegen den umlaufenden Biegekörper 5 und vorgelagert um einen Winkel von ebenfalls 270° sowie nachgelagert um einen Winkel von ca. 30° gegen den zentrischen Biegekörper 7 angelegt. Zusätzlich wird der Instrumentenkörper 1 von der auch in dieser Ausführung vorhandenen Austrittsschräge 10a um einen Winkel von ca. 30° gebogen. Insgesamt ergibt dies einen Gesamtkrümmungswinkel von ca. 600°.

Fig. 22 zeigt die Situation mit einmalig um 360° auf der Umlaufbahn 6 umgelaufenem Biegekörper 5. In diesem Fall wird der Instrumentenkörper 1 anschließend an die Eintrittsöffnung 9 zunächst von einer dortigen Eintrittsschräge 9a um ca. 35° gebogen, anschließend umgibt er den umlaufenden Biegekörper 5 zunächst um ca. 215°, den zentrischen Biegekörper 7 um 180°, den umlaufenden Biegekörper 5 nochmals um 270° und den zentrischen Biegekörper 7 nochmals um 120°. Hinzu kommt ein zusätzlicher Biegewinkel von 30° durch die Austrittsschräge 10a. Insgesamt resultiert dies in einem Gesamtkrümmungswinkel von ca. 850°.

Das Ausführungsbeispiel der Fig. 23 bis 26 unterscheidet sich von demjenigen der Fig. 19 bis 22 durch den weiteren, zweiten umlaufenden Biegekörper 5₄ zusätzlich zum ersten umlaufenden Biegekörper 5s, wobei der zweite umlaufende Biegekörper 5₄ dem ersten umlaufenden Biegekörper 5s um den Abstandwinkel Wₐ von in diesem Fall 90° in Umlaufrichtung folgt bzw. nachhinkt. Fig. 23 zeigt den Ausgangszustand mit dem Instrumentenkörper 1 in geradliniger Durchführung seines Bedienbereichs 1b durch das Bediengehäuse 8.

Fig. 24 zeigt die Situation bei um 90° auf der Umlaufbahn 6 weiter bewegten umlaufenden Biegekörpern 5s, 5₄. In dieser Situation wird der Instrumentenkörper 1 durch den ersten umlaufenden Biegekörper 5s um ca. 180° und durch den zweiten umlaufenden Biegekörper 5₄ um ca. 125° gebogen. Des Weiteren tragen die auch hier vorhandenen Schrägflächen an der Eintrittsöffnung 9 und der Austrittsöffnung 10, d.h. die Eintrittsschräge 9a und Austrittschräge 10a, mit einer weiteren Biegung des Instrumentenkörpers von ca. 35° bzw. ca. 90° bei. Daraus resultiert ein Gesamtkrümmungswinkel des Instrumentenkörpers 1 von ca. 430°.

Fig. 25 zeigt die Anordnung nach einem Umlauf der Biegekörper 5s, 5₄ um 270° auf der Umlaufbahn 6. In diesem Fall hat sich der Instrumentenkörper 1 anschließend an die Eintrittsöffnung 9 zunächst um ca. 90° gegen den zentrischen Biegekörper 7 angelegt, anschließend um 180° gegen den zweiten umlaufenden Biegekörper 5₄ und um 225° um den ersten umlaufenden Biegekörper 5s, gefolgt von einer weiteren Biegung um ca. 100° um den zweiten umlaufenden Biegekörper 5₄ und eine abschließende Biegung durch die Austrittsschräge 10a um ca. 53°. Daraus resultiert eine Gesamtkrümmung des Instrumentenkörpers 1 von ca. 648°.

Fig. 26 zeigt die Anordnung nach einem vollständigen Umlauf der beiden Biegekörper 5s, 5₄ um 360° auf der Umlaufbahn 6. Der Instrumentenkörper 1 wird in diesem Fall ausgehend von der Eintrittsöffnung 9 zunächst an der Eintrittsschräge 9a um ca. 36° gebogen, und danach sukzessive durch den ersten umlaufenden Biegekörper 5s um ca. 126°, dem zentrischen Biegekörper 7 um ca. 90°, den zweiten umlaufenden Biegekörper 5₄ um ca. 135°, den ersten umlaufenden Biegekörper 5s um ca. 225°, den zweiten Biegekörper 5₄ um 180° und abschließend durch den zentrischen Biegekörper 7 und die Austrittsschräge 10a nochmals um jeweils ca. 31°. Dies führt zu einem Gesamtbiegewinkel von ca. 854°.

Beim Ausführungsbeispiel von Fig. 27 ist der Instrumentenkörper 1, wie bereits oben kurz erwähnt, mit seinem proximalen Ende an dem um seine Längsachse 5L drehbeweglich im Bediengehäuse 8 der Bedieneinheit 4 angeordneten Biegekörper 5 umfangsseitig fixiert. Durch Drehen des Biegekörpers 5 um seine Längsachse 5L lässt sich der Instrumentenkörper 1 in seinem Bedienbereich 1b auf den Umfang des Biegekörpers 5 aufwickeln und dadurch von seinem in Fig. 27 gezeigten, geradlinigen Ausgangspositionsverlauf VA in einen entsprechend gekrümmten Verlauf biegen. In diesem Fall entspricht der Drehwinkel des Biegekörpers 5 direkt dem Gesamtbiegewinkel für den Instrumentenkörper 1.

Im Ausführungsbeispiel der Fig. 28 bis 32 sind, wie ebenfalls bereits oben kurz erwähnt, ein erster umlaufender Biegekörper 5₁ und ein zweiter umlaufender Biegekörper 5₂ diametral gegenüberliegend auf der Umlaufbahn 6 angeordnet, wobei sie zwischen sich lediglich einen Spalt zur Durchführung des Instrumentenkörpers 1 belassen. Fig. 28 zeigt die Anordnung im geradlinigen Ausgangspositionsverlauf VA des über die Eintrittsöffnung 9 in den Innenraum 8a des Bediengehäuses 8 der Bedieneinheit 4 eingeführten und über die Austrittsöffnung 10 wieder aus diesem herausgeführten Bedienbereichs 1b des Instrumentenkörpers 1.

Fig. 29 veranschaulicht die Anordnung nach einer Umlaufbewegung der beiden Biegekörper 5₁, 5₂ um 90° auf der Umlaufbahn 6. Die beiden Biegekörper 5₁, 5₂ nehmen dabei den Instrumentenkörper 1 in seinem Bedienbereich 1b mit, so dass dieser wiederum einen gekrümmten Verlauf mit mehreren bogenförmigen Krümmungsabschnitten VK_{b} erfährt. Dabei wird der Instrumentenkörper 1 ausgehend von der Eintrittsöffnung 9 zunächst an der Eintrittsschräge 9a um ca. 45° gebogen, wonach er sich um jeweils ca. 135° gegen den zweiten Biegekörper 5₂ und den ersten Biegekörper 5₁ anlegt, bevor er an der Austrittsschräge 10a nochmals um ca. 45° gebogen wird. Dies führt zu einem Gesamtkrümmungswinkel von ca. 360°.

Fig. 30 zeigt die Anordnung nach einer Umlaufbewegung der beiden Biegekörper 5₁, 5₂ um 180° bezogen auf den Ausgangszustand. Der Instrumentenkörper 1 wird hierbei durch die Eintrittsschräge 9a um ca. 45° und anschließend durch den zweiten Biegekörper 5₂ und den ersten Biegekörper 5₁ um jeweils ca. 225° und abschließend durch die Austrittsschräge 10a nochmals um ca. 45° gebogen. Dies resultiert in einem Gesamtkrümmungswinkel für den Instrumentenkörper 1 von ca. 540°.

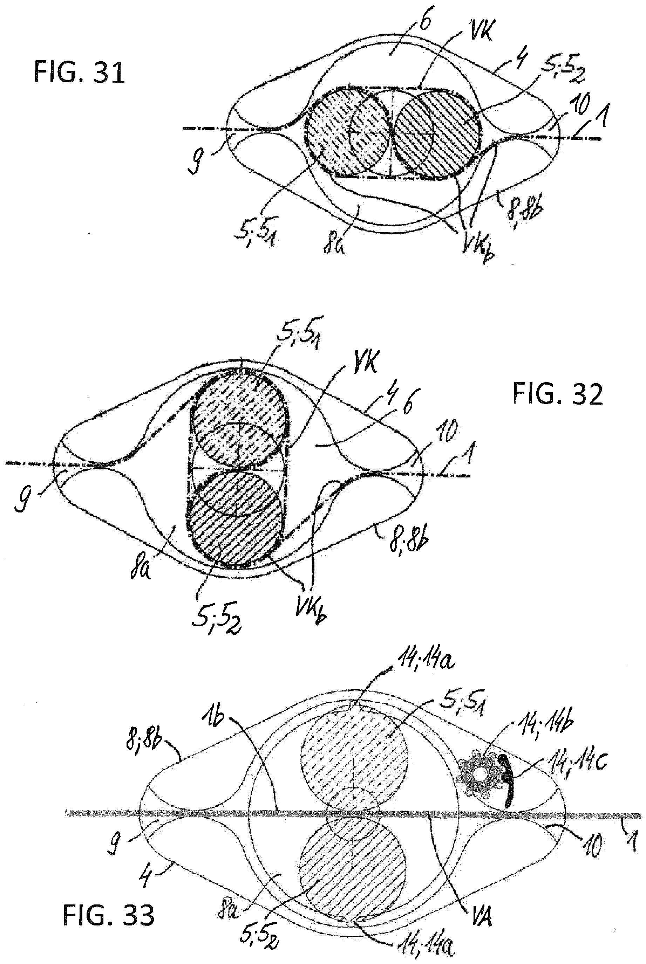

Fig. 31 zeigt die Anordnung nach einer Umlaufbewegung der beiden Biegekörper 5₁, 5₂ auf der Umlaufbahn 6 um 270°. Der Instrumentenkörper 1 wird dabei durch die Eintrittsschräge 9a um ca. 45° und anschließend sukzessive durch den ersten Biegekörper 5₁ um 45°, den zweiten Biegekörper 5₂ um 270°, den ersten Biegekörper 5₁ um 270°, den zweiten Biegekörper 5₂ um ca. 45° und durch die Austrittsschräge 10a um ca. 45° gebogen. Daraus resultiert ein Gesamtkrümmungswinkel für den Instrumentenkörper 1 von ca. 720°.

Fig. 32 zeigt die Situation nach einem vollständigen Umlauf der beiden Biegekörper 5₁, 5₂ um 360°. Der Instrumentenkörper 1 wird hierbei durch die Eintrittsschräge 9a um ca. 45° und anschließend sukzessive durch den ersten Biegekörper 5₁ um 225°, den zweiten Biegekörper 5₂ um 270°, den ersten Biegekörper 5₁ um 180°, den zweiten Biegekörper 5₂ um ca. 135° und die Austrittsschräge 10a um ca. 45° gebogen. Dies resultiert in einem Gesamtbiegewinkel des Instrumentenkörpers 1 in seinem Bedienbereich 1b von ca. 900°.

Für den Benutzer kann es hilfreich sein, wenn er über den aktuell vorliegenden Umlaufwinkel der Biegekörper 5₁, 5₂ bzw. Krümmungszustand des Instrumentenkörpers 1 im Bedienbereich 1b informiert ist. So ist es z.B. in Ausführungen, bei denen der Instrumentenkörper 1 in der Bedieneinheit 4 gewechselt werden kann, d.h. ein anderer Instrumentenkörper 1 mit der Bedieneinheit 4 gekoppelt werden kann, für den Benutzer nützlich zu wissen, ob sich der Instrumentenkörper 1 exakt oder zumindest ungefähr in seinem Ausgangspositionsverlauf VA befindet, da ein solcher Wechsel des Instrumentenkörpers 1 in diesem Zustand normalerweise einfacher und daher bevorzugt ist. Hierfür beinhaltet das Funktionsschlauchinstrument in zugehörigen Ausführungen einen Umlaufzähler 14, wie im Beispiel von Fig. 33.

Im Beispiel der Fig. 33 beinhaltet der Umlaufzähler 14 je einen Zählnocken 14a am Umfang jedes der beiden Biegekörper 5₁, 5₂ und ein Zählrad 14b, das drehbeweglich am Bediengehäuse 8 angeordnet ist. Sobald einer der Zählnocken 14a am Zählrad vorbeiläuft, was jeweils nach einem halben Umlauf der Biegekörper 5₁, 5₂ auf der Umlaufbahn 6 der Fall ist, dreht er dieses von einer aktuellen in eine nächste Rastposition, wobei ein elastisches Rastelement 14c das Zählrad 14b in seiner jeweiligen Rastposition hält. Auf diese Weise ist der Umlaufzähler 14 in dieser Realisierung in der Lage, dem Benutzer den momentanen Umlaufwinkel der Biegekörper 5₁, 5₂ bzw. Krümmungszustand des Instrumentenkörpers 1 bis auf eine halbe Umdrehung genau anzuzeigen, was für den Benutzer in aller Regel ausreichend ist. Durch entsprechend modifizierte Ausführung des Umlaufzählers 14 kann dessen Anzeigegenauigkeit bei Bedarf auch höher sein. Insbesondere kann der Umlaufzähler 14 auf Wunsch so realisiert sein, dass er sofort erkennt bzw. anzeigt, wenn der Instrumentenkörper 1 aus seinem Ausgangspositionsverlauf VA herausbewegt wurde, und ebenso trennscharf erkennt, wenn der Instrumentenkörper 1 wieder in seinen Ausgangspositionsverlauf VA zurückbewegt wurde. Dies ermöglicht dem Benutzer eine sichere Erkennung, dass bzw. ob sich der Instrumentenkörper 1 in seinem Ausgangspositionsverlauf VA befindet.

Fig. 34 veranschaulicht schematisch eine Ausführung der Bedieneinheit 4 mit zwei synchron translationsbeweglich angeordneten Biegekörpern 5₅, 5e und drei weiteren Biegekörpern 5₇, 5₈, 5₉, relativ zu denen die beiden erstgenannten Biegekörper 5₅, 5₆ längs der Verschiebungsrichtung TR verschoben, d.h. translatorisch bewegt, werden können. Zur leichteren Unterscheidung werden die beiden erstgenannten Biegekörper 5₅, 5₆ im Folgenden als erste Biegekörper und die drei anderen Biegekörper 5₇, 5s, 5₉ als zweite Biegekörper bezeichnet. In Fig. 34 ist der Ausgangszustand der betreffenden Bedieneinheit 4 mit gestrichelten Linien angedeutet, der Betätigungszustand mit durchgezogenen Linien. Wie daraus ersichtlich, ist der Ausgangspositionsverlauf VA des Instrumentenkörpers 1 im Bedienbereich 1b wiederum geradlinig, und entlang dieser Richtung, zu der die Verschiebungsrichtung TR senkrecht ist, sind zum einen die beiden ersten Biegekörper 5₅, 5e und zum anderen die drei zweiten Biegekörper 5₇, 5s, 5₉ jeweils in einer Reihe liegend voneinander beabstandet angeordnet. Um die Betätigungsbewegung der Bedieneinheit 4 zu bewirken, beinhaltet die Bedieneinheit 4 zwei in Fig. 34 nicht gezeigte Bedienteile, die relativ zueinander translatorisch bewegbar sind, wobei am einen Bedienteil die beiden ersten Biegekörper 5₅, 5₆ und am anderen Bedienteil die drei zweiten Biegekörper 5₇, 5s, 5₉ angeordnet sind.

Die Abstände bzw. Zwischenräume zwischen je zwei benachbarten ersten bzw. zweiten Biegekörpern 5₅, 5₆; 5₇, 5s, 5₉ entsprechen im gezeigten Beispiel im Wesentlichen dem Außendurchmesser der Biegekörper 5₅ bis 5s, so dass bei der Translationsbewegung der ersten Biegekörper 5₅, 5₆ relativ zu den zweiten Biegekörpern 5₇, 5₈, 5s, wie sie durch die Betätigung der Bedieneinheit 4 bewirkt wird, die ersten Biegekörper 5₅, 5₆ in einen jeweiligen Zwischenraum zwischen den zweiten Biegekörpern 5₇, 5s, 5₉ und der mittlere zweite Biegekörper 5s in den Zwischenraum zwischen den beiden ersten Biegekörpern 5₅, 5₆ hineingelangen können. Die beiden ersten Biegekörper 5₅, 5₆ nehmen hierbei den Instrumentenkörper 1 im Bedienbereich 1b in der Verschiebungsrichtung TR mit, wodurch sich dieser gleichzeitig auch gegen die zweiten Biegekörper 5₇, 5s, 5₉ anlegt.

Im Betätigungszustand, wie er in Fig. 34 exemplarisch gezeigt ist und in dem der Instrumentenkörper 1 im Bedienbereich 1b seinen Krümmungsverlauf VK annimmt, liegt der Instrumentenkörper 1 über jeweils den halben Umfang gegen die beiden ersten Biegekörper 5₅, 5e und den mittleren zweiten Biegekörper 5s und über einen Umfangswinkel von 90° gegen die beiden äußeren zweiten Biegekörper 5₇, 5₉ an. Die dafür benötigte Zusatzlänge, d.h. die Längendifferenz des Instrumentenkörpers 1 im Bedienbereich 1b zwischen diesem Krümmungsverlauf VK und dem geradlinigen Ausgangspositionsverlauf VA stammt vom in Fig. 34 rechts liegenden, proximal vorgelagerten Bereich des Instrumentenkörpers 1.

Denn die Hüllenfixierung 16 am distalen Endbereich 4a der Bedieneinheit 4 verhindert dort ein Einziehen des Instrumentenkörpers 1 in die Bedieneinheit 4 vom distal nachgelagerten Bereich des Instrumentenkörpers 1, wenn sich der Klemmstift 18 wie gezeigt in seiner Klemmposition 18a befindet, in die er aus einer rückwärtigen Löseposition vom Benutzer durch eine Vorschubkraft FV axial vorgeschoben werden kann. Dies gewährleistet, dass ein am distalen Endbereich des Instrumentenkörpers 1 angeordnetes Nutzelement an seinem gedachten Einsatzort verbleibt und nicht axial zurückgezogen wird. Lediglich der sich durch die Krümmungslängendifferenz von Hülle 2 und Kern 3 ergebende Axialhub AH wird zum distalen Funktionsbereich 1a übertragen, da die Hüllenfixierung 16 bzw. der Klemmstift 18 in seiner Klemmposition 18a nur die Hülle 2 des Instrumentenkörpers 1 an der Bedieneinheit 4 festlegt, hingegen eine Axialbewegung des innenliegenden Kerns 3 zulässt.

Da der Kern 3 im proximalen Fixierbereich 1c des Instrumentenkörpers 1 an der Hülle 2 gegen axiale Relativbewegung gesichert fixiert ist, wird er gemeinsam mit der Hülle 2 vom vorgelagerten proximalen Abschnitt des Instrumentenkörpers 1 her in die Bedieneinheit 4 eingezogen, wenn dort der Instrumentenkörper 1 mit seinem Bedienbereich 1b durch die Betätigung des Bedienelements 4 von seinem kürzeren Ausgangspositionsverlauf VA in seinen längeren Krümmungsverlauf VK verbracht wird.

Es versteht sich, dass in entsprechend modifizierten Ausführungen nur ein oder mehr als zwei erste Biegekörper sowie nur ein oder zwei oder mehr als drei zweite Biegekörper vorgesehen sein können, die jeweils in Längsrichtung des eingebrachten oder einzubringenden Instrumentenkörpers 1 voneinander beabstandet angeordnet sind.

Im Beispiel von Fig. 34 sind zum einen die ersten Biegekörper 5₅, 5e und zum anderen die zweiten Biegekörper 5₇, 5s, 5₉ jeweils ohne Versatz in der Verschiebungsrichtung TR angeordnet. Fig. 35 zeigt eine modifizierte Ausführung, bei der die beiden ersten Biegekörper 5₅, 5₆ in der Verschiebungsrichtung TR um eine vorgebbare Versatzlänge LV gegeneinander versetzt angeordnet sind. Dies hat zur Folge, dass der bezüglich der Verschiebungsrichtung TR nach hinten verlagerte erste Biegekörper 5e bei Betätigung der Bedieneinheit 4 mit dem entsprechenden Versatz erst später als der vordere erste Biegekörper 5₅ gegen den Instrumentenkörper 1 zur Anlage kommt und sich nicht wie der vordere erste Biegekörper 5s mit seinem gesamten Durchmesser in den Zwischenraum zwischen den beiden betreffenden zweiten Biegekörpern 5s, 5₉ hineinbewegt. Im gezeigten Beispiel von Fig. 35 entspricht die Versatzlänge LV etwa dem Radius der in diesem Fall sämtlich gleich groß gewählten zylindrischen Biegekörper 5₅ bis 5₉.

Die in der Verschiebungsrichtung TR versetzte Anordnung der ersten Biegekörper 5₅, 5₆ ermöglicht eine besonders feinfühlige Einstellung und Veränderung des Axialhubs AH, da zunächst nur der vordere erste Biegekörper 5₅ den Axialhub AH bewirkt und erst mit weiterer Betätigung der Bedieneinheit 4 der hintere erste Biegekörper 5e zum Bewirken des Axialhubs AH beiträgt.

Fig. 36 zeigt eine Ausführungsvariante des Beispiels von Fig. 34, bei der die Biegekörper 5₅ bis 5₉ mit Ausnahme des distal letzten zweiten Biegekörpers 5₇ drehbeweglich am jeweiligen Bedienteil gehalten sind, wie durch Drehpfeile symbolisiert. Bei Betätigung des Bedienelements 4 können sich daher die drehbeweglichen Biegekörper 5₅, 5₆, 5₈, 5₉ beim Anlegen gegen den Instrumentenkörper 1 und Mitnehmen des Instrumentenkörpers 1 im Bedienbereich 1b verdrehen, so dass es nicht erforderlich ist, dass der Instrumentenkörper 1 an der Kontaktoberfläche des jeweiligen Biegekörpers 5₅, 5₆, 5₈, 5₉ entlanggleiten muss. Der distal letzte zweite Biegekörper 5₇ ist hingegen bei dieser Ausführung drehfest an der Bedieneinheit 4 gehalten und repräsentiert den bereits oben erwähnten unbewegten Biegekörper 19 der in diesem Fall so gebildeten Hüllenfixierung 16 und fungiert folglich als Alternative zum Klemmstift 18 im Beispiel von Fig. 34. Der Instrumentenkörper 1 kann sich gegen die feststehende Oberfläche des drehfesten Biegekörpers 19 mit Reibschluss anlegen, wobei der Reibschluss groß genug gewählt ist, dass er ein Entlanggleiten des Instrumentenkörpers 1 mit dessen Hülle 2 verhindert. Bei Bedarf kann hierzu die Oberfläche des drehfesten Biegekörpers 19 geeignet reibschlusserhöhend ausgebildet sein, z.B. durch Aufrauen.

Fig. 36 veranschaulicht die Wirkung dieser Hüllenfixierung 16. In einem oberen Teilbild ist die Bedieneinheit 4 im Ausgangszustand gezeigt, in einem unteren Teilbild im Betätigungszustand. Im Betätigungszustand besitzt der Instrumentenkörper 1 im Bedienbereich 1b mit seinem Krümmungsverlauf VK eine größere Länge als im geradlinigen Ausgangspositionszustand VA, wie bereits oben zu Fig. 34 erläutert. Aufgrund der Fixierung der Hülle 2 durch die Hüllenfixierung 16 am distalen Endbereich 4a der Bedieneinheit 4 wird der Instrumentenkörper 1 um die betreffende Zusatzlänge von seinem vorgelagerten proximalen Bereich her in die Bedieneinheit 4 eingezogen, d.h. der Instrumentenkörper 1 wird um eine entsprechende Länge dp von seinem proximalen Bereich her eingezogen. Ohne die Hüllenfixierung 16 würde das Einziehen des Instrumentenkörpers 1 in die Bedieneinheit 4 zwecks Bereitstellung der Zusatzlänge für seinen Bedienbereich 1b grundsätzlich auch vom distal nachgelagerten Bereich des Instrumentenkörpers 1 her erfolgen, d.h. der Instrumentenkörper 1 würde sich dann um eine gewisse Länge dd an seinem distalen Ende axial zurückbewegen. Durch die Hüllenfixierung 16 wird diese distale Einzugslänge dd verhindert, d.h. die distale Einzugslänge dd hat dann den Wert null, wie in Fig. 36 illustriert. Dies hat, wie bereits erwähnt, die in den meisten Anwendungen erwünschte Folge, dass sich die axiale Lage des Nutzelements im distalen Funktionsbereich 1a des Instrumentenkörpers 1 bei Betätigung der Bedieneinheit 4 zwecks Bereitstellung des Axialhubs AH nicht ändert.

Die Figuren 37 bis 40 veranschaulichen detaillierter eine Ausführung der Bedieneinheit 4, welche auf dem Prinzip des Ausführungsbeispiels von Fig. 34 beruht und diesem mit der Modifikation entspricht, dass ein weiterer erster Biegekörper 5₁₀ vorgesehen ist und alle Biegekörper 5₅ bis 5₁₀ drehbeweglich an der Bedieneinheit 4 gehalten sind, wie oben zu den Biegekörpern 5₅, 5₆, 5₈, 5₉ des Ausführungsbeispiels von Fig. 36 erläutert.

Beim Ausführungsbeispiel der Figuren 37 bis 40 beinhaltet die Bedieneinheit 4 zwei gegeneinander translatorisch längs der Verschiebungsrichtung TR bewegbare Bedienteile 4₁, 4₂, die gleichzeitig entsprechend translationsbewegliche Gehäuseteile 8b, 8c des dadurch gebildeten Bediengehäuses 8 der Bedieneinheit 4 bilden. Die drei ersten Biegekörper 5₅, 5₆, 5₁₀ sind am einen Bedienteil 4₁ und die drei zweiten Biegekörper 5₇, 5₈ 5₉ am anderen Bedienteil 4₂ drehbeweglich gehalten. Die drehbewegliche Lagerung erfolgt über entsprechende Achsstummel 20, wie insbesondere aus den Fig. 39 und 40 ersichtlich. In dieser gezeigten Ausführung ist der Klemmstift 18 am Bedienteil 4₂ bzw. dem dadurch gebildeten Gehäuseteil 8b im distalen Endbereich 4a der Bedieneinheit 4 angeordnet, wobei er in Fig. 37 in seiner axial vorgeschobenen Klemmposition 18a und in Fig. 38 in seiner axial zurückgezogenen Löseposition 18b gezeigt ist.

Wenn sich der Klemmstift 18 in seiner Löseposition 18b befindet, kann der Instrumentenkörper 1 von der Eintrittsöffnung 9 oder der Austrittsöffnung 10 her in das Bediengehäuse 8 eingeschoben und durch dieses hindurchgeschoben werden, wobei er an der gegenüberliegenden Austrittsöffnung 10 bzw. Eintrittsöffnung 9 wieder aus dem Bediengehäuse 8 austritt. Durch Verbringen des Klemmstifts 18 in seine Klemmposition 18a wird dann der Instrumentenkörper 1 mit seiner Hülle 2 am Bediengehäuse 8 axial fixiert. Wenn der Instrumentenkörper 1 wieder vom Bediengehäuse 8 und damit der Bedieneinheit 4 abgenommen werden soll, braucht lediglich der Klemmstift 18 wieder in seine Löseposition 18b verbracht werden, wonach die Bedieneinheit 4 vom Instrumentenkörper 1 abgezogen werden kann.

Die Realisierung der Hüllenfixierung 16 mit dem Klemmstift 18 ermöglicht es dem Benutzer, den Instrumentenkörper 1 durch eine einfache Bedienbetätigung des Klemmstifts 18 wahlweise an der Bedieneinheit 4 zu fixieren und diese Fixierung wieder zu lösen, indem er den Klemmstift 18 zwischen seiner Löseposition 18b und seiner Klemmposition 18a verlagert. Dies kann bei dieser und funktionell analogen Realisierungen der Hüllenfixierung 16 bei Bedarf sehr vorteilhaft vom Benutzer für ein bequemes und zuverlässiges sukzessives Vorschieben bzw. Einführen des Instrumentenkörpers 1 in eine Kanüle z.B. eines Katheters oder direkt in einen Körpergewebekanal genutzt werden. Dazu platziert der Benutzer die Bedieneinheit 4 beim oder nach dem Anbringen am Instrumentenkörper 1 zunächst mit einem gewissen, nicht zu großen Abstand vom distalen Ende des Instrumentenkörpers 1, indem er den Klemmstift 18 in seine Löseposition 18b verbringt und die Bedieneinheit 4 entlang des Instrumentenkörpers 1 entsprechend verschiebt. Danach bringt er den Klemmstift 18 in seine Klemmposition 18a, wonach der Benutzer sehr bequem den Instrumentenkörper 1 an der Bedieneinheit 4 halten und ihn mit seinem distalen Ende in eine Zugangsöffnung der Kanüle bzw. des Gewebekanals einführen und anschließend darin vorschieben kann, bis er mit der Bedieneinheit 4 nahe vor die Zugangsöffnung kommt. Daraufhin verbringt er den Klemmstift 18 wieder in seine Löseposition 18b. Nun kann er die Bedieneinheit 4 am Instrumentenkörper 1 um ein gewünschtes Maß zurückschieben. Anschließend bringt der Benutzer den Klemmstift 18 wieder in seine Klemmposition 18a und schiebt die Bedieneinheit 4 um das zuvor zurückgeschobene Maß vor, wobei nun der an der Bedieneinheit 4 fixierte Instrumentenkörper 1 der Vorschubbewegung folgt und sich dadurch entsprechend weiter in die Kanüle bzw. den Gewebekanal vorbewegt. Dieser Vorgang wird wiederholt, bis der Instrumentenkörper 1 weit genug vorgeschoben wurde. Da der Benutzer zum Vorschieben auf diese Weise die relativ nahe zur Zugangsöffnung positionierbare Bedieneinheit 4 nutzen kann, braucht er hierzu nicht den dünnen und häufig relativ glatten und deshalb im Allgemeinen schwieriger handhabbaren Instrumentenkörper 1 direkt handhaben. Zum Schluss kann der Benutzer bei Bedarf die Bedieneinheit 4 an die für den anschließenden Gebrauch zur Bereitstellung des Axialhubs AH gewünschte Position auf dem Instrumentenkörper 1 verschieben.

Das Bedienteil 4₂ bzw. Gehäuseteil 8b besitzt in dieser exemplarischen Ausführung einen U-förmigen Querschnitt, wie aus den Fig. 39 und 40 ersichtlich, und das Bedienteil 4₁ bzw. Gehäuseteil 8c bildet einen quaderförmigen Drucktastenkörper, der von der offenen U-Seite des Bedienteils 4₂ bzw. Gehäuseteils 8b in den Aufnahmeraum der U-Form eingefügt und in diesem längs der Verschiebungsrichtung TR translationsbeweglich geführt ist.

Wie insbesondere aus Fig. 39 ersichtlich, sind die zylindrischen Biegekörper 5₅ bis 5₁₀ umfangsseitig mit einer jeweiligen Führungsrille 21 zum erleichterten Führen des dagegen zur Anlage kommenden Instrumentenkörpers 1 versehen.

Ein großer Vorteil liegt bei Realisierungen der Bedieneinheit 4 nach Art der Ausführungsbeispiele der Fig. 15 bis 26 und 28 bis 40 darin, dass der Benutzer vor betriebsgemäßem Gebrauch des Instruments den Instrumentenkörper 1 an der Bedieneinheit 4 nicht gegen Axialbewegung gesichert aufwändig als Ganzes fixieren muss, wie dies bei herkömmlichen Endoskopieinstrumenten dieser Art üblicherweise erforderlich ist. Vielmehr braucht der Benutzer den Instrumentenkörper 1 nur lose durch das Gehäuse 8 der Bedieneinheit 4 hindurchführen oder ihn in anderer, an sich bekannter Weise lose mit der Bedieneinheit 4 koppeln sowie bei Bedarf die Hülle 2 mittels der Hüllenfixierung 16 in einfacher Weise am distalen Endbereich 4a der Bedieneinheit 4 festlegen. Beim Ausführungsbeispiel von Fig. 27 kann eine axiale Fixierung des Instrumentenkörpers 1 an der Bedieneinheit 4 in ebenfalls relativ einfacher Weise am um seine Längsachse 5L drehbewegten Wickelkörper 5_{w} durch eine zugehörige form- und/oder kraftschlüssige Verbindung erfolgen.

Die Figuren 41 und 42 veranschaulichen schematisch eine Ausführungsvariante des Beispiels von Fig. 34, bei der die ersten Biegekörper 5₅, 5₆ einerseits und die zweiten Biegekörper 5₇, 5s, 5₉ andererseits relativ zueinander um die Schwenkachse 17 schwenkbeweglich statt translationsbeweglich angeordnet sind. Die in diesem Fall relativ zueinander schwenkbeweglich angeordneten Bedienteile 4₁, 4₂ bzw. Gehäuseteile 8b, 8c sind in den Figuren 41 und 42 der Einfachheit halber nur symbolisch angedeutet. Die ersten Biegekörper 5₅, 5e sind mit radialem Abstand auf der Verbindungslinie ihrer Mittelpunkte zur Schwenkachse 17 am Bedien- bzw. Gehäuseteil 4_{1,} 8c angeordnet, die zweiten Biegekörper 5₇, 5s, 5₉ mit radialem Abstand auf der Verbindungslinie ihrer Mittelpunkte zur Schwenkachse 17 am Bedien- bzw. Gehäuseteil 4₂, 8b. Durch die Schwenkbetätigung der Bedieneinheit 4, wie in Fig. 42 durch einen Schwenkpfeil SP angedeutet, gelangen die ersten Biegekörper 5₅, 5₆ wiederum in die Zwischenräume zwischen je zwei benachbarten der zweiten Biegekörper 5₇, 5₈, 5₉, und der mittlere zweite Biegekörper 5s in den Zwischenraum zwischen den beiden ersten Biegekörpern 5₅, 5e, wie aus Fig. 42 ersichtlich, welche wiederum die Bedieneinheit 4 mit gestrichelten Linien im Ausgangszustand und mit durchgezogenen Linien im Betätigungszustand schematisch darstellt.

Da die ersten bzw. die zweiten Biegekörper 5₅ bis 5₉ jeweils mit radialem Abstand voneinander angeordnet sind, wird in diesem Beispiel durch die Schwenkbetätigung der Bedieneinheit 4 ein analoger Effekt wie beim Ausführungsbeispiel von Fig. 35 mit den translationsbeweglichen und gegeneinander versetzt angeordneten ersten Biegekörpern 5s, 5₆ erzielt, wonach zunächst nur der eine der beiden ersten Biegekörper 5₅, 5e und erst später der andere erste Biegekörper 5e gegen den Instrumentenkörper 1 im Bedienbereich 1b zur Anlage kommt und diesen in seiner Bewegung mitnimmt. Dies resultiert wiederum in der oben zum Ausführungsbeispiel von Fig. 35 erläuterten, sehr feinfühligen Veränderbarkeit bzw. Einstellbarkeit des durch die Betätigung der Bedieneinheit 4 bereitgestellten Axialhubs AH.

Es versteht sich, dass die Erfindung weitere Ausführungsformen mit zwei oder beliebig mehr bewegten Biegekörpern umfasst, die auf einer Umlaufbahn umlaufend oder translationsbeweglich oder schwenkbeweglich derart angeordnet sind, dass sie bei Betätigung der Bedieneinheit 4 sukzessive nacheinander statt gleichzeitig biegend bzw. krümmend auf den Instrumentenkörper 1 im Bedienbereich 1b einwirken, was wie gesagt die Feinfühligkeit der Einstellung des Axialhubs AH steigern kann.

Wie die gezeigten und die weiteren oben erläuterten Ausführungsbeispiele deutlich machen, stellt die Erfindung in vorteilhafter Weise ein Funktionsschlauchinstrument zur Verfügung, bei dem sich ein Nutzelement im distalen Funktionsbereich durch einen Axialhub betätigen lässt, der vom Benutzer über die Bedieneinheit sehr feinfühlig, exakt und bedienfreundlich bewirkt bzw. eingestellt werden kann, vorzugsweise stufenlos. Insbesondere lassen sich damit z.B. für endoskopische Funktionsschlauchinstrumente, wie Führungsdrähte und Katheterinstrumente, bei üblichen Dimensionierungen solcher Instrumente relativ geringe Hublängen für den Axialhub im Bereich von einem oder wenigen Millimetern oder nur einem oder einige Zehntel Millimeter exakt einstellen. Aber auch größere Hublängen im Bereich von mehreren Millimetern lassen sich problemlos realisieren, indem der Instrumentenkörper in einen entsprechend größeren Gesamtkrümmungswinkel gebogen wird, beispielsweise durch Biegen in mehrere vollständige Wicklungen um jeweils 360° oder durch Verwenden einer geeigneten Mehrzahl von z.B. translationsbeweglichen oder schwenkbeweglichen oder auf einer Umlaufbahn umlaufend beweglichen Biegekörpern. Wie oben erläutert, hängt der Axialhub von der Dimensionierung des Instrumentenkörpers ab, insbesondere von der Wandstärke der Hülle und dem Durchmesser bzw. der Wandstärke des Kerns, so dass sich z.B. bei gleichem Gesamtkrümmungswinkel ein größerer Axialhub für ein Instrument mit größerer Wandstärke der Hülle bzw. größerem Kerndurchmesser ergibt.

Als weiterer besonderer Vorteil ermöglicht die Erfindung Ausführungen des Funktionsschlauchinstruments, bei denen es nicht erforderlich ist, dass der Benutzer vor betriebsgemäßem Gebrauch des Instruments den Instrumentenkörper an der Bedieneinheit gegen Axialbewegung gesichert in einer aufwändigen Weise fixieren muss. Vielmehr kann es genügen, dass der Benutzer den Instrumentenkörper einfach nur lose durch ein Gehäuse der Bedieneinheit hindurchführt oder ihn in anderer Weise lose mit der Bedieneinheit koppelt, was ihm die Handhabung deutlich vereinfachen und den Nutzerkomfort erhöhen kann. Bei Bedarf kann er durch die Hüllenfixierung für eine einfache, lösbare Festlegung der Hülle des Instrumentenkörpers an einem distalen Endbereich der Bedieneinheit und damit für eine Konstanthaltung der Lage der Hülle im distalen Endbereich des Instruments bei Betätigung der Bedieneinheit zwecks Erzeugung des gewünschten Axialhubs des Kerns relativ zur Hülle sorgen.

## Patentansprüche

1. Funktionsschlauchinstrument, insbesondere endoskopisches Funktionsschlauchinstrument, mit
- einem schlauchartig langgestreckten Instrumentenkörper (1), der eine biegefähige Hülle (2) und einen sich in dieser erstreckenden, biegefähigen Kern (3) aufweist und dafür eingerichtet ist, eine axiale Relativbewegung von Hülle (2) und Kern (3) und dadurch einen Axialhub (AH) mindestens in einem distalen Funktionsbereich (1a) zur Betätigung eines Nutzelements ermöglichen, und
- einer Bedieneinheit (4), mit welcher der Instrumentenkörper (1) an einem dem distalen Funktionsbereich (1a) proximal vorgelagerten Bedienbereich (1b) zur Bewirkung des Axialhubs (AH) gekoppelt ist,
**dadurch gekennzeichnet, dass**
- die Bedieneinheit (4) dafür eingerichtet ist, bei Betätigung den Instrumentenkörper (1) im Bedienbereich (1b) reversibel von einem Ausgangspositionsverlauf (VA) in einen gegenüber diesem stärker gekrümmten Krümmungsverlauf (VK) zu biegen, der eine den Axialhub (AH) bereitstellende Krümmungslängendifferenz von Hülle (2) und Kern (3) entlang des Krümmungsverlaufs (VK) bewirkt.

2. Funktionsschlauchinstrument nach Anspruch 1, weiter **dadurch gekennzeichnet, dass**
- die Hülle (2) einen Schraubenfederkörper (2f) aus einem Runddrahtmaterial (2fr) oder einem Flachdrahtmaterial (2ff) beinhaltet und/oder
- der Kern (3) einen Massivdrahtkörper (3m) oder einen Hohldrahtkörper (3h) beinhaltet.

3. Funktionsschlauchinstrument nach Anspruch 1 oder 2, weiter **dadurch gekennzeichnet, dass** der Krümmungsverlauf (VK) einen oder mehrere bogenförmige Abschnitte (VK_{b}) und/oder einen oder mehrere Vollwicklungen (VK_{w}) umfasst.

4. Funktionsschlauchinstrument nach einem der Ansprüche 1 bis 3, weiter **dadurch gekennzeichnet, dass** die Bedieneinheit (4) in einem distalen Endbereich (4a) eine die Hülle (2) gegen Axialbewegung sichernde Hüllenfixierung (16) aufweist.

5. Funktionsschlauchinstrument nach einem der Ansprüche 1 bis 4, weiter **dadurch gekennzeichnet, dass** die Bedieneinheit (4) mindestens einen bewegten Biegekörper (5) aufweist, gegen dessen Umfang sich der Instrumentenkörper (1) in seinem Krümmungsverlauf (VK) anlegt.

6. Funktionsschlauchinstrument nach Anspruch 5, weiter **dadurch gekennzeichnet, dass**
- der bewegte Biegekörper (5) um seine Längsachse (5L) drehbeweglich ist und den Instrumentenkörper (1) zum Verbringen in seinen Krümmungsverlauf (VK) aufwickelt oder
- der bewegte Biegekörper (5) auf einer Umlaufbahn (6) umläuft und den Instrumentenkörper (1) zum Verbringen in seinen Krümmungsverlauf (VK) mitnimmt oder
- der bewegte Biegekörper (5) um eine Schwenkachse (17) verschwenkbar ist und den Instrumentenkörper (1) zum Verbringen in seinen Krümmungsverlauf (VK) mitnimmt oder
- der bewegte Biegekörper (5) längs einer Verschiebungsrichtung (TR) translationsbeweglich ist und den Instrumentenkörper (1) zum Verbringen in seinen Krümmungsverlauf (VK) mitnimmt.

7. Funktionsschlauchinstrument nach Anspruch 6, weiter **dadurch gekennzeichnet, dass** sich die Umlaufbahn (6) um einen zentrischen Biegekörper (7) herum erstreckt, gegen dessen Umfang sich der Instrumentenkörper (1) in seinem Krümmungsverlauf (VK) anlegt.

8. Funktionsschlauchinstrument nach Anspruch 6 oder 7, weiter **dadurch gekennzeichnet, dass** die Bedieneinheit (4) mindestens einen ersten und einen zweiten bewegten Biegekörper (5₁, 5₂; 5s, 5₄; 5₅, 5s) aufweist, gegen deren Umfang sich der Instrumentenkörper (1) in seinem Krümmungsverlauf (VK) anlegt und die mit umlaufseitigem Abstand auf der Umlaufbahn (6) umlaufen oder translationsbeweglich mit unterschiedlichem Abstand quer zur Verschiebungsrichtung (TR) oder schwenkbeweglich mit radialem Abstand zur Schwenkachse (17) angeordnet sind und den Instrumentenkörper (1) zum Verbringen in seinen Krümmungsverlauf (VK) mitnehmen.

9. Funktionsschlauchinstrument nach Anspruch 8, weiter **dadurch gekennzeichnet, dass** sich der erste und der zweite Biegekörper (5₁, 5₂) diametral auf der Umlaufbahn (6) gegenüberliegen und zwischen sich den Instrumentenkörper (1) führen.

10. Funktionsschlauchinstrument nach Anspruch 8, weiter **dadurch gekennzeichnet, dass** der erste und der zweite Biegekörper (5s, 5₄) um einen Umlaufbahnwinkel (Wa) kleiner als 180°, insbesondere zwischen 80° und 100°, voneinander beabstandet sind und um den zentrischen Biegekörper (7) umlaufen.

11. Funktionsschlauchinstrument nach Anspruch 6, weiter **dadurch gekennzeichnet, dass** die Bedieneinheit (4) zwei gegeneinander verschwenkbare oder translatorisch bewegbare Bedienteile (4₁, 4₂) aufweist, an denen jeweils ein oder mehrere Biegekörper (5₅ bis 5₉) angeordnet sind, wobei zwischen zwei Biegekörpern (5s, 5₉) des einen Bedienteils (4₂) ein Zwischenraum zum Eingreifen eines Biegekörpers (5₆) des anderen Bedienteils (4₁) belassen ist.

12. Funktionsschlauchinstrument nach einem der Ansprüche 5 bis 11, weiter **dadurch gekennzeichnet, dass** die Bedieneinheit (4) ein Bediengehäuse (8) mit einem Gehäuseinnenraum (8a) aufweist, das den mindestens einen bewegten Biegekörper (5) und den Bedienbereich (1b) des Instrumentenkörpers (1) aufnimmt, wobei das Bediengehäuse (8) eine Eintrittsöffnung (9) in den Gehäuseinnenraum (8a) und eine Austrittsöffnung (10) aus dem Gehäuseinnenraum (8a) für den Instrumentenkörper (1) aufweist.

13. Funktionsschlauchinstrument nach Anspruch 12, weiter **dadurch gekennzeichnet, dass** das Bediengehäuse (8) zwei relativ zueinander drehbewegliche oder schwenkbewegliche oder translationsbewegliche Gehäuseteile (8b, 8c) umfasst, wobei die Eintrittsöffnung (9) und die Austrittsöffnung (10) am einen Gehäuseteil (8b) und der mindestens eine bewegte Biegekörper (5) am anderen Gehäuseteil (8c) angeordnet sind.
